(19) Europäisches Patentamt
European Patent Office
Office européen des brevets

(11) Numéro de publication: **0 510 165 B1**

# (12) FASCICULE DE BREVET EUROPEEN

(45) Date de publication de fascicule du brevet: **25.01.95**

(51) Int. Cl.$^{6}$: **C07C 43/11**, C07C 43/178, C07C 43/23, C07D 317/22, A61K 7/48

(21) Numéro de dépôt: **91920912.2**

(22) Date de dépôt: **13.11.91**

(86) Numéro de dépôt internationale : **PCT/FR91/00889**

(87) Numéro de publication internationale : **WO 92/08685 (29.05.92 92/12)**

(54) **COMPOSES AMPHIPHILES NON-IONIOUES DERIVES DU GLYCEROL, LEUR PROCEDE DE PREPARATION, COMPOSES INTERMEDIAIRES CORRESPONDANTS ET COMPOSITIONS CONTENANT LESDITS COMPOSES.**

(30) Priorité: **14.11.90 FR 9014149**
**08.08.91 FR 9110128**

(43) Date de publication de la demande: **28.10.92 Bulletin 92/44**

(45) Mention de la délivrance du brevet: **25.01.95 Bulletin 95/04**

(84) Etats contractants désignés: **AT BE CH DE DK ES FR GB GR IT LI LU NL SE**

(56) Documents cités:
**EP-A- 0 066 107**
**EP-A- 0 071 019**
**EP-A- 0 283 165**
**GB-A- 2 144 122**

(73) Titulaire: **L'OREAL**
**14, Rue Royale**
**F-75008 Paris (FR)**

(72) Inventeur: **ZYSMAN, Alexandre**
**6, rue George-Eastman**
**F-75013 Paris (FR)**
Inventeur: **SEBAG, Henri**
**22, rue Erlanger**
**F-75016 Paris (FR)**
Inventeur: **RIBIER, Alain**
**2, boulevard Jourdan**
**F-75014 Paris (FR)**
Inventeur: **VANLERBERGHE, Guy**
**40, rue du Général-de-Gaulle**
**Montjay-la-Tour**
**F-77410 Villevaude (FR)**
Inventeur: **MAHIEU, Claude**
**90, avenue de Villiers**
**F-75017 Paris (FR)**
Inventeur: **BERTHELOT, Claude**
**155, allée du Colonel Fabien**
**F-93320 Les Pavillons-sous-Bois (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

(74) Mandataire: **Michardière, Bernard et al**
**Cabinet Peuscet**
**68, rue d'Hauteville**
**F-75010 Paris (FR)**

## Description

L'invention concerne des composés amphiphiles non-ioniques dérivés du glycérol comportant plusieurs chaînes lipophiles, leur procédé de préparation, des composés intermédiaires de la préparation desdits composés amphiphiles obtenus dans ledit procédé de préparation et des compositions contenant lesdits composés.

On connaît déjà, par les brevets français 1 477 048 et 1 484 723, des composés amphiphiles non-ioniques du glycérol ne comportant qu'une chaîne lipophile fixée sur une chaîne hydrophile et qui ont pour formules :

$$(A) \quad R_1O \left[ C_2H_3O\ (CH_2OH) \right]_n H$$

dans laquelle $R_1$ est un radical alkyle et n est un nombre inférieur à 10, et

$$(B) \quad R_2\text{-}O \left[ C_2H_3O\ (CH_2OCH_2\text{-}CHOH\text{-}CH_2OH) \right]_n H$$

où $R_2$ est un radical alkyle et n est un nombre inférieur ou égal à 5.

On connait également par le brevet français 2 482 128 des composés amphiphiles non-ioniques dérivés du glycérol comportant deux chaînes lipophiles, qui ont pour formule :

(C) 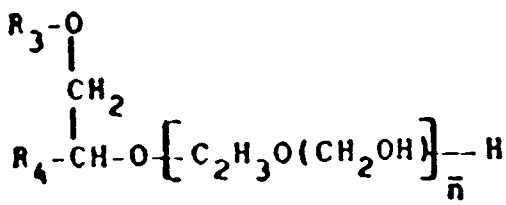

où $R_3$ et $R_4$ peuvent être des radicaux alkyle et $\bar{n}$ est une valeur statistique moyenne comprise entre 2 et 20.

Ces composés connus peuvent être utilisés comme agents tensioactifs, comme agents émulsifiants ou, dans le cas des composés de formules (A) et (C), pour la fabrication de vésicules non-ioniques.

On connait, par l'article MURAMATSU et SCHMID (Chem. Phys. Lipids 1972,9(2), p.123-132), le composé de formule (E) comportant une seule chaîne lipophile :

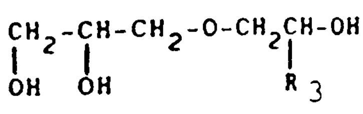 (E)

formule dans laquelle $R_3$ est un radical hydrocarboné en $C_{14}$ ou $C_{15}$.

La présente invention concerne des composés amphiphiles non-ioniques dérivés du glycérol, caractérisés par le fait qu'ils ont la formule (I) :

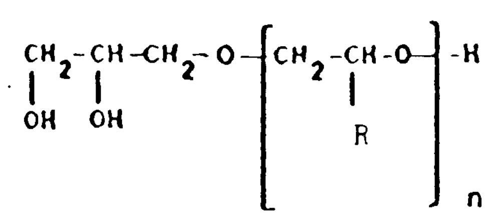 (I)

formule dans laquelle R représente un radical pris dans le groupe formé par les radicaux alkyle ou alcényle, linéaires ou ramifiés en $C_4$-$C_{28}$ et leurs mélanges, ou représente un groupement-$CH_2A$ dans lequel A représente -OR',-SR' ou

$$-O-\overset{\overset{O}{\|}}{C}-R',$$

R' représentant un radical hydrocarboné saturé ou insaturé, et n représente une valeur statistique moyenne $\bar{n}$ supérieure à 1 et au plus égale à 6 et, lorsque R est -$CH_2A$, n est également une valeur égale à 2. Le radical hydrocarboné R' est de préférence un radical alkyle linéaire en $C_8$ -$C_{22}$, un radical alkyle ramifié en $C_8$ - $C_{36}$, un radical alcényle en $C_{18}$ ou un radical alkylaryle à chaîne alkyle linéaire ou ramifié en $C_8$ - $C_{16}$ ; dans le radical alkylaryle, le groupe aryle est, de préférence, un groupe phényle ; le radical alcényle est, avantageusement un radical octadécène-9 yle ou octadécanediène-9,12 yle. Lorsque R est -$CH_2A$, A est de préférence OR'.

Des composés de formule (I) préférés sont ceux où R représente un radical alkyle linéaire en $C_{14}$ - $C_{18}$ ou un groupement -$CH_2A$ dans lequel A est OR', R' représentant un radical linéaire en $C_{10}$ -$C_{18}$, et n représente une valeur statistique moyenne $\bar{n}$ supérieure à 1 et au plus égale à 3, et, lorsque R représente -$CH_2A$, est également une valeur égale à 2.

La présente invention a également pour objet la préparation des composés de formule (I) par un procédé en deux stades avec formation de produit(s) intermédiaire(s), caractérisé par le fait que :

- dans un premier stade, on fait réagir, en présence d'un catalyseur basique, de l'isopropylidèneglycérol de formule (IV) avec un époxyde de formule (III) dans lequel R a la même signification que dans la formule (I), pour obtenir un (des) produit(s) intermédiaire(s) de formule (II) selon le schéma de réaction suivant :

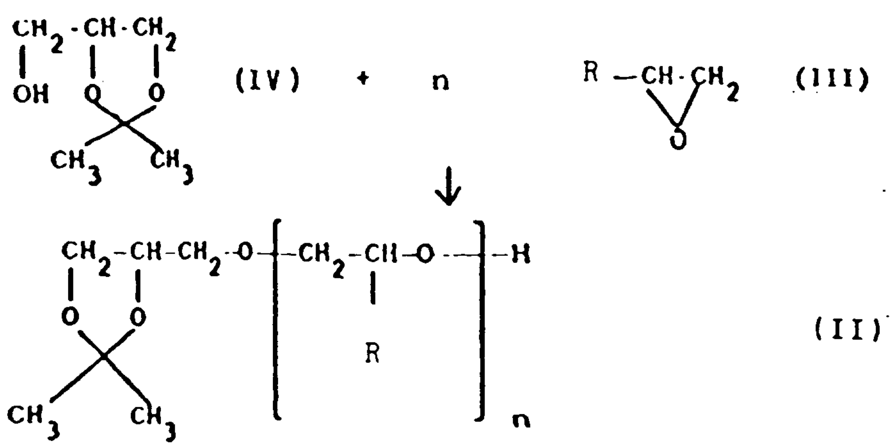

(IV) + n (III)

(II)

R et n ayant la même signification que dans la formule (I)
et

- dans un second stade, on hydrolyse le(les) produit(s) intermédiaire(s) de formule (II) obtenu(s) et l'on sépare le(les) composé(s) de formule (I) du mélange réactionnel.

Au premier stade du procédé de préparation, le catalyseur basique utilisé est, de préférence, choisi dans le groupe formé par les métaux alcalins, les hydrures de métaux alcalins, les hydroxydes alcalins, les alcoolates alcalins, les amines, les fluorures de métaux alcalins tels que KF, RbF, CsF, ces fluorures étant de préférence absorbés sur alumine. On utilise, de préférence, un alcoolate alcalin, en particulier le tert-butylate de potassium. La quantité de catalyseur basique utilisée est, avantageusement, comprise entre 0,5 et 100 %, de préférence entre 4 et 40 %, en moles par rapport à l'isopropylidèneglycérol de formule (IV).

Au premier stade, on opère, de préférence, de la manière décrite ci-après. On mélange d'abord au moins une partie de l'isopropylidèneglycérol de formule (IV) et le catalyseur basique sous atmosphère inerte, par exemple sous atmosphère d'azote, et on chauffe à une température comprise entre 50 et 190 °C, de préférence voisine de 150 °C. Le mélange est, de préférence, effectué en l'absence de solvant, mais des solvants comme le diméthylformamide ou la méthylpyrrolidone peuvent être utilisés.

Dans le mélange obtenu, on ajoute l'époxyde de formule (III), éventuellement dissous dans le reste d'isopropylidèneglycérol de formule (IV). Cette addition peut s'effectuer soit d'un coup, soit progressivement, généralement sur une période comprise entre 30 minutes et 2 heures.

Au second stade, le (les) produit(s) intermédiaire(s) obtenu(s) de formule (II) est (sont) ensuite hydrolysé(s). Cette hydrolyse est, de préférence, effectuée en présence d'un catalyseur acide. Celui-ci peut être un acide minéral comme les acides chlorhydrique, fluorhydrique, sulfurique ou phosphorique ou un acide organique comme l'acide acétique ou oxalique. La réaction d'hydrolyse est, de préférence, effectuée à une température comprise entre 25 °C et 100 °C.

Un solvant peut être utilisé pour effectuer la réaction d'hydrolyse. Ce solvant est, par exemple, le méthanol, l'éthanol, l'isopropanol, l'heptane, l'hexane, l'acétone, des éthers tels que l'éther éthylique ou isopropylique ou les mono- ou diéthers de glycol tels que le diglyme.

Après la réaction d'hydrolyse, le mélange est filtré, puis le filtrat est chauffé sous pression réduite pour éliminer les produits volatils et recueillir le(les) composé(s) de formule (I).

La présente invention a également pour objet, à titre de produit industriel nouveau, les composés non-ioniques intermédiaires de formule (II) obtenus au premier stade du procédé de préparation des composés de formule (I).

Les composés de formule I selon l'invention peuvent être utilisés pour la préparation de compositions cosmétiques ou pharmaceutiques, en particulier dermopharmaceutiques.

Tous les composés de formule I sont des tensioactifs. Ils peuvent donc être utilisés comme tels dans les compositions. Ils peuvent, par conséquent, être utilisés comme agents dispersants, agents émulsionnants ou agents de lavage.

La présente invention a donc pour objet une composition cosmétique ou pharmaceutique contenant au moins un composé de formule I utilisé comme tensioactif. On peut utiliser comme agent tensioactif un ou plusieurs composés de formule I ou on peut les utiliser en association avec des agents tensioactifs classiques.

De façon connue, les compositions cosmétiques ou pharmaceutiques peuvent être des compositions huileuses sous forme liquide, de gel ou de solides d'aspect cireux. Dans ces compositions, les composés de formule I sont avantageusement utilisés comme dispersants.

Ces compositions peuvent également être des émulsions de type eau-dans-l'huile ou huile-dans-l'eau dans lesquelles les composés de formule I peuvent être utilisés comme agent émulsionnant. On peut associer un ou plusieurs composés de formule I à d'autres agents émulsionnants classiques tels que des acides gras ou des alcools gras polyoxyéthylénés, des alkyléthers de polyglycérol, des esters d'acide gras et de sorbitan polyoxyéthylénés ou non, des esters d'acide gras et de sorbitol polyoxyéthylénés ou non, l'huile de ricin polyoxyéthylénée, des sels d'acides gras et d'amines ou de métaux polyvalents, des alkylsulfates polyoxyéthylénés ou non et des alkylphosphates polyoxyéthylénés ou non.

De façon connue, la phase grasse des émulsions utilisées en cosmétique ou en pharmacie contient essentiellement des huiles ou des cires. Parmi les huiles on peut citer, de façon non limitative, les huiles minérales telles que la vaseline, les huiles animales telles que les huiles de baleine, de phoque, de foie de flétan, de foie de morue, de thon, de vison, les huiles végétales telles que les huiles d'amande, d'arachide, de germe de blé, de maïs, d'olive, de jojoba, de sésame et de tournesol et les huiles de silicone. Parmi les cires on peut citer de façon non limitative la cire de sipol, la lanoline, la lanoline hydrogénée, la lanoline acétylée, la cire d'abeille, la cire de candellila, la cire microcristalline, la paraffine, la cire de carnauba, le spermaceti, le beurre de cacao, le beurre de karité, les cires de silicone et les huiles hydrogénées concrètes à 25 °C. Les huiles et les cires peuvent également être choisies parmi les esters d'acides gras en $C_{12}$ à $C_{22}$, saturés ou insaturés et d'alcools ou polyols inférieurs comme l'isopropanol, le glycol ou le glycérol, ou d'alcools gras en $C_8$ -$C_{22}$, linéaires ou ramifiés, saturés ou insaturés, ou encore d'alcane diols -1,2 en $C_{10}$ - $C_{22}$.

Les compositions dans lesquelles les composés de formule I sont utilisés comme tensioactifs contiennent de préférence de 0,5 à 50 % et plus généralement de 0,5 à 25 % en poids de composé de formule I par rapport au poids total de la composition.

Dans les compositions selon l'invention, les produits ou mélanges de composés (I) peuvent être associés à d'autres agents de surface ioniques ou non-ioniques, à des polymères naturels ou synthétiques, ioniques ou non-ioniques, à des huiles ou des cires, à des protéines plus ou moins hydrolysées, à des épaississants, à des nacrants, à des émollients, à des hydratants, à des colorants, à des agents réducteurs ou oxydants, à des conservateurs, à des parfums, à des filtres anti-UV, à des solvants, à des propulseurs ou à des produits actifs pharmaceutiques ou parapharmaceutiques.

Certains composés de formule (I), en particulier ceux où R représente un radical alkyle linéaire en $C_{14}$ - $C_{18}$ ou représente -$CH_2$A, A étant -OR' et R' représentant un radical alkyle linéaire en $C_{10}$ - $C_{18}$, et n représente une valeur statistique moyenne $\bar{n}$ supérieure à 1 et au plus égale à 3, et, lorsque R représente -$CH_2$A, est également égal à 2, sont des lipides amphiphiles non-ioniques capables de former des

vésicules à structure lamellaire.

De façon connue, ces vésicules sont caractérisées par une structure en feuillets constitués de couches de phase lipidique encapsulant une phase aqueuse.

Ces vésicules sont, de façon connue, préparées sous forme de dispersion dans une phase aqueuse. On trouvera une liste non limitative de divers modes de préparation dans "Les liposomes en biologie celullaire et pharmacologie" Editions INSERM/John Libbey Eurotext, 1987, pages 6 à 18.

Les vésicules obtenues sont donc constituées d'une phase lipidique constituée d'un ou plusieurs feuillets encapsulant une phase E et elles sont dispersées dans une phase aqueuse de dispersion D.

Les vésicules formées avec les composés de formule (I) pour lesquels R représente un radical alkyle linéaire en $C_{14}$ - $C_{18}$ ou représente -$CH_2$A, A étant -OR' et R' représentant un radical alkyle linéaire en $C_{10}$ - $C_{18}$, et n représente une valeur statistique moyenne $\bar{n}$ supérieure à 1 et au plus égale à 3, et, lorsque R représente -$CH_2$A, est également égal à 2, possèdent dans l'ensemble un bon taux de gonflement, une faible perméabilité et une bonne stabilité. De façon surprenante, les composés préférés de formule (I) pour lesquels R représente un groupe alkyle linéaire en $C_{14}$ ou un groupe -$CH_2$A, A étant -OR' et R' représentant un groupe alkyle linéaire en $C_{16}$ permettent d'obtenir, en présence d'un véhicule cosmétique, des dispersions de vésicules ayant une viscosité plus élevée que celle obtenue en utilisant les composés de formule (A) définis ci-dessus comportant une seule chaîne lipophile. En effet, il est connu que les actifs cosmétiques couramment introduits dans les phases vésiculaires ont un effet fluidifiant et engendrent une chute de viscosité des compositions.

L'utilisation de ces dispersions de vésicules obtenues avec les composés de formule (I) préférés permet donc de préparer des crèmes épaisses, riches en principes actifs, en limitant l'effet fluidifiant des actifs cosmétiques.

La présente invention a donc également pour objet une composition cosmétique ou pharmaceutique contenant, dispersées dans une phase aqueuse D, des vésicules délimitées par un ou plusieurs feuillets formés d'une phase lipidique contenant au moins un composé de formule (I) dans laquelle R représente un radical alkyle linéaire en $C_{14}$ - $C_{18}$, de préférence un radical alkyle linéaire en $C_{14}$, ou représente -$CH_2$A, A étant OR' et R' représentant un radical alkyle linéaire en $C_{10}$ - $C_{18}$, de préférence un radical alkyle linéaire en $C_{16}$, et n représente une valeur statistique moyenne $\bar{n}$ supérieure à 1 et au plus égale à 3, et, lorsque R est -$CH_2$A est également égal à 2.

De façon connue, on peut associer dans la phase lipidique, aux lipides amphiphiles non-ioniques de formule (I), d'autres lipides amphiphiles ioniques et/ou lipides amphiphiles non-ioniques.

Les lipides amphiphiles ioniques, qui peuvent être mélangés dans la phase lipidique aux lipides amphiphiles de formule (I) sont, de préférence, choisis dans le groupe formé par les phospholipides naturels, modifiés par voie chimique ou enzymatique, ou de synthèse, les composés anioniques et les gangliosides.

Parmi les phospholipides naturels, on peut citer la lécithine d'oeuf ou de soja, et la sphingomyéline ; parmi les phospholipides de synthèse, on peut citer la dipalmitoyl-phosphatidylcholine et parmi les phospholipides modifiés, on peut citer la lécithine hydrogénée.

Parmi les composés anioniques, on peut citer ceux qui sont représentés par la formule :

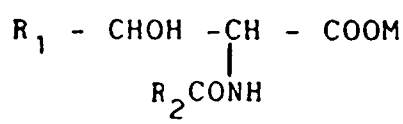

formule dans laquelle :

$R_1$ représente un radical alkyle ou alcényle en $C_7$ - $C_{21}$ ;

$R_2$ représente un radical hydrocarboné, saturé ou insaturé, en $C_7$ - $C_{31}$ ; et

M représente H, Na, K, $NH_4$ ou un ion ammonium substitué dérivé d'une amine.

Les lipides amphiphiles non-ioniques, qui peuvent être mélangés dans la phase lipidique aux lipides amphiphiles sont, de préférence, choisis dans le groupe formé par :

(1) les dérivés de polyglycérol, linéaires ou ramifiés, de formule :

$$R_3O - \left[ C_3H_5\ (OH)O \right]_{\bar{n}} - H$$

dans laquelle :
$-C_3H_5(OH)O-$ est représenté par les structures suivantes prises en mélange ou séparément : $-CH_2CHOHCH_2O-$ ;

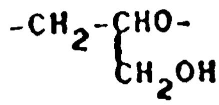
;
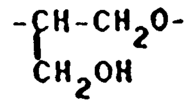

$\bar{n}$ est une valeur statistique moyenne comprise entre 2 et 6 ;
$R_3$ représente :

(a) une chaîne aliphatique, linéaire ou ramifiée, saturée ou insaturée, contenant de 12 à 30 atomes de carbone ; ou les radicaux hydrocarbonés des alcools de lanoline ;
(b) un reste $R'_3CO$, où $R'_3$ est un radical aliphatique, linéaire ou ramifié, en $C_{11}$ - $C_{17}$ ;
(c) un reste

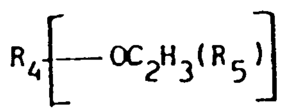

où

. $R_4$ peut prendre la signification (a) ou (b) donnée pour $R_3$ ;
. $-OC_2H_3(R_5)-$ est représenté par les structures suivantes, prises en mélange ou séparément :

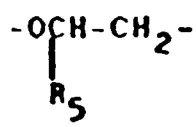
et
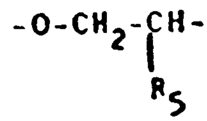

où $R_5$ prend la signification (a) donnée pour $R_3$ ;

(2) les éthers de polyglycérol, linéaires ou ramifiés, comportant deux chaînes grasses ;
(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétique ;
(7) les hydroxyamides représentés par la formule :

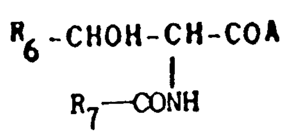

dans laquelle :

- $R_6$ désigne un radical alkyle ou alcényle en $C_7$ - $C_{21}$ ;
- $R_7$ désigne un radical hydrocarboné, saturé ou insaturé, en $C_7$ - $C_{31}$ ;
- COA désigne un groupement choisi parmi les deux groupements suivants :
  . un reste

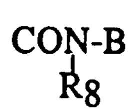

où

B est un radical dérivé d'amines primaires ou secondaires, mono- ou polyhydroxylées ; et

$R_8$ désigne un atome d'hydrogène ou un radical méthyle, éthyle ou hydroxyéthyle ; et

. un reste -COOZ, où Z représente le reste d'un polyol en $C_3$ -$C_7$.

Dans les dérivés de polyglycérol, le radical $R_3$ aliphatique linéaire saturé est, de préférence, un radical lauryle, myristyle, cétyle, stéaryle, arachidyle, béhényle, lignocéryle ou un mélange de ces radicaux ; et le radical $R_3$ aliphatique non saturé est avantageusement un radical palmitoléyle, oléyle, linoléyle, arachidonyle.

Les composés définis au point (3) ci-dessus sont avantageusement des alcools en $C_{12}$ - $C_{22}$ portant 2 à 20 unités oxyde d'éthylène (O.E.). Le stérol est, avantageusement le cholestérol ; il peut être substitué par 2 à 20 unitées d'O.E. De même, le phytostérol peut être substitué par 2 à 20 moles d'O.E.

Les éthers de polyols définis au point (4) ci-dessus sont, de préférence, des alkyl éthers de polyol en $C_2$ - $C_7$.

Les esters de polyols définis au point (5) ci-dessus sont avantageusement des esters de sorbitol.

Les glycolipides utilisables comme lipides amphiphiles non-ioniques sont avantageusement les cérébrosides.

On peut avantageusement incorporer, de façon connue, dans la phase lipidique au moins un additif, qui permet de diminuer la perméabilité des vésicules, et/ou au moins un lipide chargé destiné à améliorer la stabilité des vésicules, en prévenant leur floculation et leur fusion, et à permettre l'augmentation du taux d'encapsulation.

On peut notamment citer parmi ces additifs ou lipides chargés utilisables :

- les stérols et leurs dérivés, par exemple oxyéthylénés, plus particulièrement le cholestérol, le sulfate de cholestérol acide et ses sels alcalins et le phosphate de cholestérol acide et ses sels alcalins ;
- les alcools et diols à longue chaîne ;
- les amines à longue chaîne et leurs dérivés ammonium quaternaire ;
- les dihydroxyalkylamines ;
- les amines grasses polyoxyéthylénées ;
- les esters d'aminoalcools à longue chaîne et leurs sels et dérivés ammonium quaternaire ;
- les esters phosphoriques d'alcools gras, par exemple le dicétylphosphate et le dimyristylphosphate sous forme acides ou de sels alcalins;

Les compositions obtenues avec les composés selon l'invention peuvent contenir, de façon connue, un ou plusieurs composé(s) actif(s) ayant une activité cosmétique et/ou dermopharmaceutique, qui, selon leurs caractéristiques de solubilité, peuvent avoir différentes localisations. Par exemple, dans le cas des dispersions de vésicules contenant une phase aqueuse encapsulée, si les actifs sont liposolubles, on les introduit dans la phase lipidique constituant le(s) feuillet(s) des vésicules ; si les actifs sont hydrosolubles, on les introduit dans la phase aqueuse encapsulée des vésicules ; si les actifs sont amphiphiles, ils se répartissent entre la phase lipidique et la phase aqueuse encapsulée avec un coefficient de partage, qui varie selon la nature de l'actif amphiphile et les compositions respectives de la phase lipidique et de la phase aqueuse encapsulée. De façon générale, les actifs sont mis en place dans la phase lipidique des feuillets et/ou dans la phase encapsulée par les feuillets.

Dans le cas des émulsions de type huile-dans-l'eau ou eau-dans-l'huile contenant un composé de formule I comme agent émulsionnant, les composés liposolubles sont introduits dans la phase huileuse et les composés hydrosolubles dans la phase aqueuse. De même, les actifs amphiphiles se répartissent entre la phase aqueuse et la phase huileuse.

Des actifs hydrosolubles sont, par exemple, la glycérine, le sorbitol, l'érythrulose et les antibiotiques ; des actifs liposolubles sont, par exemple, l'acide rétinoïque, les lipoprotides et les stéroïdes.

Les actifs introduits peuvent avoir des activités cosmétiques et/ou dermopharmaceutiques (ou "fonctions") très variables qui sont données dans le tableau ci-après.

# TABLEAU

| FONCTION | ACTIFS UTILISABLES |
|---|---|
| Anti-oxydant ou anti-radicaux libres. | Les extraits des plantes suivantes :<br>- Aubépine.<br>- Ginkgo biloba.<br>- Thé vert.<br>- Vigne.<br>- Romarin<br>Les enzymes :<br>- Commercialisées sous la dénomination SB 12 par SEDERMA et constituées par un mélange de lactoferrine et de lactoperoxydase, de glucose oxydase et de thiocyanate de potassium.<br>- Superoxyde dismutase.<br>- Glutathion peroxydase.<br>La superphycodismutase extraite d'algues.<br>Les coenzymes Q, en particulier la coenzyme Q10.<br>Les séquestrants, en particulier des dérivés d'acides polyphosphoniques.<br>Les tanins.<br>Le sélénium et ses dérivés, en particulier la séléniométhionine.<br>Les peptides, par exemple un mélange d'extraits de rate et de thymus,<br>Thiolim et sérum albumine bovine non stabilisée.<br>Les protéines, par exemple l'hémocyanine qui est une protéine cuivrée extraite de l'escargot marin et l'apohemocyanine qui est une protéine analogue sans cuivre.<br>Les flavonoïdes, notamment la catéchine, les proanthocyanidines, les flavanols, les flavones, les isoflavones, les flavanénols, les flavanones, les flavanes et les chalcones.<br>Les caroténoïdes, notamment le ß-carotène et le rocou.<br>L'acide sorbohydroxyamique.<br>Les tocophérols, notamment l'alpha-tocophérol et l'acétate d'alpha-tocophérol.<br>Le palmitate d'ascorbyle.<br>Le gallate de propyle.<br>L'acide caféique et ses dérivés.<br>L'acide ascorbique.<br>L'acide homogentisique.<br>L'acide érythorbique.<br>L'acide nordihydroguaïacétique.<br>Le laurylméthionate de lysine.<br>Le butylhydroxyanisole.<br>Le butylhydroxytoluène.<br>Les substances "SOD like". |

TABLEAU (suite 1)

| FONCTION | ACTIFS UTILISABLES |
|---|---|
| Hydratant ou humectant | Une reconstitution de sueur ("Normal moisturizing factors"-NMF).<br>Le pyroglutamate de sodium.<br>L'acide hyaluronique.<br>Les dérivés de chitosane (carboxyméthylchitine).<br>Le ß-glycérophosphate.<br>Le lactamide.<br>L'acétamide.<br>Le lactate d'éthyle, de sodium et de triéthanolamine.<br>Le pyrrolidone carboxylate de métaux en particulier de Mg, Zn, Fe ou Ca.<br>La thiamorpholinone.<br>L'acide orotique.<br>Les acides carboxyliques alpha-hydroxylés en $C_3$ à $C_{20}$, notamment l'acide alpha-hydroxy propionique.<br>Les polyols, notamment l'inositol, le glycérol, la diglycérine, le sorbitol.<br>Les polyolosides, notamment alginate et guar.<br>Les protéines, notamment le collagène soluble et la gélatine.<br>Les lipoprotides choisis parmi les dérivés mono ou polyacylés d'acides aminés ou de polypeptides dans lesquels le reste acide RCO comporte une chaîne hydrocarbonée en $C_{13}$-$C_{19}$, notamment l'acide palmitoylcaséïnique, l'acide palmitoyl collagénique, le dérivé dipalmitoyl-O-N de l'hydroxyproline, le stéaroyl glutamate de sodium, le stéaroyl tripeptide de collagène, l'oléyltétra et pentapeptide de collagène, le linoléate d'hydroxyproline.<br>L'urée et ses dérivés, notamment la méthylurée.<br>L'extrait de tissu cutané, notamment celui commercialisé sous la dénomination "OSMODYN" par les Laboratoires Serobiologiques de Nancy (LSN) et contenant des peptides, des acides aminés, des saccharides et 17% de mannitol.<br>Plus particulièrement une association de glycérol, d'urée et d'acide palmitoylcaséïnique. |
| Mélanorégulateur :<br>1) accélérateur de bronzage | Les huiles de bergamote et de citrus.<br>L'alpha-MSH et ses homologues synthétiques.<br>La caféine.<br>Les dérivés de tyrosine, notamment le tyrosinate de glucose et la N-malyltyrosine. |

TABLEAU (suite 2)

| FONCTION | ACTIFS UTILISABLES |
|---|---|
| 2) Dépigmentant | L'acide ascorbique ou vitamine C et ses dérivés, notamment l'ascorbyle phosphate de Mg.<br>Les hydroxyacides, notamment l'acide glycolique.<br>L'acide kojique.<br>L'arbutine et ses dérivés.<br>L'hémocyanine (protéine cuivrée de l'escargot marin) et l'apohémocyanine (protéine analogue à la précédente sans cuivre).<br>L'hydroquinone et ses dérivés, notamment le monoalkyl éther et le benzyléther. |
| Coloration de la peau (brunissage artificiel) | L'ortho diacétylbenzène.<br>Les indoles.<br>La dihydroxyacétone.<br>L'érythrulose.<br>Le glycéraldéhyde.<br>Les gamma-dialdéhydes, notamment l'aldéhyde tartrique. |
| Liporégulateurs (amincissant et anti-acné, anti-séborrhée) | Les complexes de vitamines et d'oligo-éléments, notamment le complexe vitamine $B_6$/zinc.<br>L'orizanol.<br>L'acide azélaïque.<br>Les xanthines et alkylxanthines, notamment l'extrait de cola, la caféine et la théophylline.<br>L'adénosine monophosphate cyclique et non cyclique.<br>L'adénosine triphosphate.<br>L'extrait de lierre.<br>L'extrait de marron d'Inde.<br>Les extraits d'algues, notamment l'extrait d'algues rouges (fucus serratus) et le cytofiltrat.<br>L'extrait de ginseng.<br>L'extrait de Centella Asiatica (asiaticoside) contenant de la génine et de l'acide asiatique.<br>La thioxolone (HBT).<br>La S-carboxyméthylcystéine.<br>La S-benzylcystéamine. |

TABLEAU (suite 3)

| FONCTION | ACTIFS UTILISABLES |
|---|---|
| Antivieillissement et anti-rides | Les insaponifiables par exemple de soja et d'avocats.<br>Les acides gras insaturés, notamment l'acide linoléique et l'acide linolénique.<br>Les hydroxyacides, notamment l'acide glycolique.<br>Les facteurs de croissance.<br>Les complexes oligoéléments - vitamines, notamment $B_6$-Zn.<br>L'acide n-octanoyle-5 salicylique.<br>L'adénosine.<br>Le rétinol et ses dérivés, notamment l'acétate de rétinol et le palmitate de rétinol.<br>Les rétinoïdes, notamment les acides rétinoïques cis ou trans et ceux décrits dans les brevets FR-A-2 570 377 ; EP-A-199636 ; EP-A- 325540 et EP-A-409728.<br>L'association de rétinoides et de xanthines.<br>L'hydroxyproline.<br>Les acides sialiques.<br>L'extrait de rate, de thymus, Thiolim et sérum albumine bovine non stabilisé vendu sous la dénomination commerciale "SILAB" par la Société "SILAB".<br>Un extrait animal placentaire, notamment l'extrait embryonnaire placentaire de bovidés dans l'eau à 5,5 % stabilisé par 0,2 % d'exyl K100a (matrix).<br>Les protéoglycannes, en particulier le protéoglycanne de cartilage de trachée de bovidés à 5 % stabilisé (protéodermin).<br>Le colostrum.<br>Les facteurs d'oxygénation cellulaire, notamment l'octacosamol. |
| Anti-UV | Les filtres UV, notamment le paraméthoxycinnamate d'éthyl-2 hexyle ;<br>la benzophénone,<br>le benzylidène-camphre et leurs dérivés, en particulier, la tétrahydroxy-2,2', 4,4'-benzophénone et l'acide hydroxy-2 méthoxy-4 benzophénone-5 sulfonique ;<br>l'acide paraaminobenzoïque,<br>le salicylate de dipropylèneglycol,<br>l'octyl salicylate,<br>les dérivés de dibenzoylméthane vendus sous les marques EUSOLEX 8020 ou PARSOL 1789 et<br>les produits vendus sous les marques EUSOLEX 232, UNIVUL T 150, UNIVUL N 539, ESCALOL 507. |

TABLEAU (suite 4)

| FONCTION | ACTIFS UTILISABLES |
|---|---|
| Kératolytique | L'acide salicylique et ses dérivés, tels que les acides alkylsalicyliques, notamment l'acide n-octanoyl-5 salicylique et le n-dodécanoyl-5, salicylate de N-hexadécyl pyridinium.<br>L'acide rétinoïque.<br>Les enzymes protéolytiques, notamment la trypsine, l'alpha-chymotrypsine, la papaïne, la bromelaine et la pepsine.<br>Le peroxyde de benzoyle.<br>L'urée.<br>Les alpha-hydroxyacides. |
| Emollient | Esters tels que l'adipate d'isopropyle. |
| Anti-inflammatoire | Les corticoïdes tels que le 17-acétate de ß-méthasone, l'indométhacine, le ketoprofène, l'acide flufenamique, l'ibuprofene, le dichlofenac, le diflunisal, le fenclofenac, le naproxene, le piroxidam, et le sulindac.<br>Le monostéaryl éther de glycérol (alcool batylique) et le mono-cétyléther de glycérol (alcool chimylique).<br>L'acide glycyrrhétinique et ses sels, notamment d'ammonium.<br>L'alpha-bisabolol (extrait de camomille).<br>La shikonine.<br>Des extraits de plantes, tels qu'eau de bleuet, arnica, aloès.<br>Des extraits de tissu méristématique, notamment l'extrait de racine de chêne.<br>Le plancton. |
| Rafraichissant | Le menthol.<br>Le lactate de menthyle. |
| Cicatrisant | L'arbre de peau, extrait de mimosa tenui flora.<br>L'extrait de Centella Asiatica.<br>L'acide ß-glycyrrhétinique.<br>L'hydroxyproline.<br>L'arginine.<br>Un extrait placentaire.<br>Un extrait de levure.<br>Le fagaramide.<br>La N-acétyl hydroxyproline.<br>L'acide acexamique et ses dérivés. |

TABLEAU (suite 5)

| FONCTION | ACTIFS UTILISABLES |
|---|---|
| Protecteur vasculaire | Les flavonoïdes, notamment les dérivés de rutine, tels que l'étoxazorutine et le rutine propylsulfonate de sodium.<br>Les extraits végétaux, notamment l'extrait huileux de Ginkgo biloba, l'extrait de marron d'Inde (escine), de lierre (saponines) et de petit houx.<br>Le nicotinate d'alpha-tocophérol. |
| Anti-bactérien, antifongique | Le bromure de triméthylcétylammonium.<br>L'acide sorbique.<br>Le peroxyde de benzoyle.<br>Le chlorure de cétylpyridinium.<br>Le chlorure de benzalkonium.<br>L'acide parahydroxybenzoïque et ses sels.<br>Le bromo-2 nitro-2 propanediol-1,3.<br>Le trichloro-3,4,4'-carbanilide.<br>Le trichloro-2,4,4'-hydroxy-2 diphényléther.<br>L'acide déhydroacétique.<br>Un extrait de pamplemousse dans la glycérine et le propylène glycol.<br>La chlorhexidine.<br>L'hexetidine.<br>L'hexamidine. |
| Agent insectifuge | Le diméthyltoluamide. |
| Antiperspirant | Le chlorhydrate d'aluminium.<br>Le chlorure d'aluminium.<br>Le complexe lactate de sodium/aluminium chlorhydroxy.<br>Le chlorhydrate de zirconyle. |

TABLEAU (suite 6)

| FONCTION | ACTIFS UTILISABLES |
|---|---|
| Déodorant | L'oxyde de zinc.<br>Le ricinoléate de zinc.<br>L'éthyl-2 hexanediol-1,3.<br>L'hexachlorophène.<br>Le produit vendu sous la marque "IRGASAN DP 300". |
| Anti-pelliculaire | L'octopyrox.<br>Les omadines.<br>Le goudron de houille.<br>L'hydroxy-1 méthyl-4 triméthyle-2,4,4 pentyl-6 pyridinone-2.<br>Le sulfure de sélénium. |
| Anti-chute des cheveux | Les inhibiteurs de glucuronidases.<br>Les muccopolysaccharides.<br>Le nicotinate de méthyle ou d'hexyle.<br>La forskaline.<br>Le minoxidil.<br>Les xanthines.<br>Les rétinoïdes. |
| Colorant capillaire | Les bases et coupleurs d'oxydation.<br>Les colorants directs.<br>Les colorants auto-oxydables. |
| Agent décolorant pour cheveux | Le peroxyde d'hydrogène. |
| Réducteur pour permanentes | L'acide thioglycolique.<br>La cystéine.<br>La cystéamine.<br>La N-acétyl cystéine.<br>La N-acétyl cystéamine.<br>Le thioglycolate de glycérol. |
| Agent conditionneur pour peau et cheveux | Polymères cationiques, cations. |

On peut inclure un ou plusieurs des actifs, définis ci-dessus, les différents actifs pouvant être tous lipophiles, hydrosolubles ou amphiphiles ou appartenir à au moins deux de ces catégories. Les actifs introduits peuvent avoir la même fonction ou des fonctions différentes. Il faut noter que certains actifs ont plusieurs fonctions.

Dans la composition contenant des vésicules dispersées dans une phase aqueuse, la phase aqueuse de dispersion des vésicules peut également contenir au moins un actif hydrosoluble et/ou au moins un actif amphiphile.

La phase aqueuse de dispersion peut contenir, de façon connue, une dispersion de gouttelettes d'un liquide non miscible à l'eau, que les vésicules stabilisent. Par conséquent, en présence de vésicules de lipides amphiphiles ioniques et/ou de lipides amphiphiles non-ioniques, il n'est pas nécessaire d'introduire un émulsionnant usuel.

Selon la présente invention, le liquide non miscible à l'eau, qui peut être présent sous forme de dispersion dans la phase aqueuse de dispersion, est constitué par tout liquide non miscible à l'eau connu de façon générale pour pouvoir être introduit dans la phase aqueuse de dispersion de vésicules de lipides ioniques ou non-ioniques ; il peut notamment être choisi dans le groupe formé par :

- une huile animale ou végétale formée par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tricaprocaprylate de glycérol, ou une huile végétale ou animale de formule $R_9$ $COOR_{10}$, formule dans laquelle $R_9$ représente le reste d'un acide gras supérieur comportant de 7 à 19 atomes de carbone et $R_{10}$ représente une chaîne hydrocarbonée ramifiée contenant de 3 à 20 atomes de carbone, par exemple l'huile de purcellin ;
- des huiles essentielles, naturelles ou synthétiques, telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, et de cade ;
- des hydrocarbures, tels que l'hexadécane et l'huile de paraffine ;
- des carbures halogénés, notamment des fluorocarbures tels que des fluoroamines, par exemple la perfluorotributylamine, des hydrocarbures fluorés, par exemple le perfluorodécahydronaphtalène, des fluoroesters et des fluoéthers ;
- des silicones, par exemple les polysiloxanes, les polydiméthylsiloxanes et les fluorosilicones ;
- des esters d'acide minéral et d'un alcool ;
- des éthers et des polyéthers.

Le liquide non miscible à l'eau peut contenir un ou plusieurs actifs lipophiles.

Les compositions selon l'invention peuvent aussi contenir, de façon connue, des additifs de formulation n'ayant ni activité cosmétique, ni activité dermopharmaceutique propre, mais utiles pour la formulation des compositions sous forme de lotion, crème ou sérum. Ces additifs sont, en particulier, pris dans le groupe formé par les gélifiants, les polymères, les conservateurs, les colorants, les opacifiants et les parfums. Parmi les gélifiants utilisables, on peut citer les dérivés de cellulose tels que l'hydroxyéthylcellulose, les dérivés d'algues tels que le satiagum, des gommes naturelles, telles que l'adragante, et des polymères synthétiques, en particulier les mélanges d'acides polycarboxyvinyliques commercialisés sous la dénomination commerciale "CARBOPOL" par la société GOODRICH. Ces additifs sont plus particulièrement ajoutés dans une phase aqueuse, par exemple la phase aqueuse de dispersion de vésicules ou la phase aqueuse d'une émulsion huile-dans-l'eau ou eau-dans-l'huile.

Les exemples donnés ci-après, à titre illustratif et nullement limitatif, permettront de mieux comprendre l'invention.

Dans ces exemples ci-après, les époxydes de formule (III) sont connus et décrits notamment dans BEILSTEIN HANDBOOK of ORGANIC CHEMISTRY (4ème édition, Fifth Supplementary Series, Volume 7 - part 3, EV 17/1, pages 11 et 12 et pages 159 à 170.

Les éthers d'alkyle (ou alkylène) et de glycidyle (ou oxiranes d'alkyl (ou alkylène) oxyméthyle) ont été préparés, de façon connue, par action d'épichlorhydrine sur l'alcool correspondant en présence de $BF_3$ puis neutralisation par une solution aqueuse de soude (voir ULBRICHT et al., COLLECT. CZECH. CHEM. COMM. 29 (1964), pages 1466, 1467, 1473).

Les époxydes de formule (III) pour lesquels R est un radical alkyle, peuvent notamment être préparés à partir des oléfines par réaction avec l'acide monoperoxyphtalique selon EDDY et al. (Journal of America Oil Chemical Society, 40, (1963), page 92). Ces produits sont aussi disponibles industriellement purs ou en mélanges, notamment auprès des sociétés PEROXID CHEMIE G.m.b.H. (Munich) ou VIKING CHEMICAL COMPANY (Minneapolis).

Dans les exemples donnés ci-après n représente une valeur statistique moyenne et n une valeur exacte.

EXEMPLES 1 A 20 : PREPARATION DE COMPOSES DE FORMULE I

EXEMPLE 1 : PREPARATION DU COMPOSE DE FORMULE

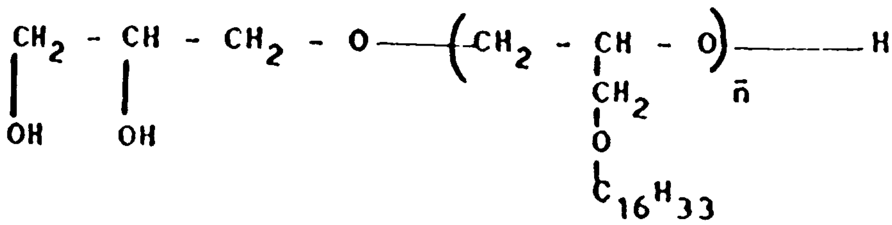

où $\bar{n}$ = 2.

Le mode opératoire est le suivant :

1er stade :

On introduit dans un réacteur 2,3 g (0,02 mole) de tert-butylate de potassium et on le dissout dans 19,5 g (0,145 mole) d'isopropylidèneglycérol en chauffant le mélange à 150 °C sous azote. On introduit ensuite à vitesse constante en deux heures 86,43 g (0,29 mole) d'éther d'hexadécyle et de glycidyle ou oxirane d'hexadécyloxyméthyle de formule :

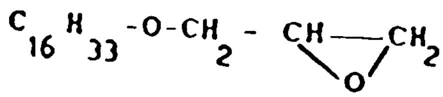

fondu en maintenant la température à 150 °C. Après la fin de l'addition, on poursuit le chauffage pendant 2 heures.

Après neutralisation et lavage à l'eau, on obtient un mélange brut contenant un produit intermédiaire de formule :

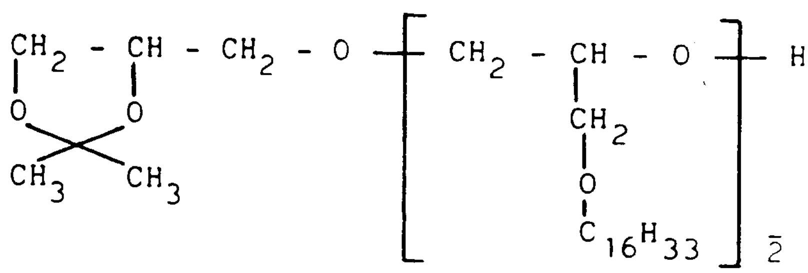

qui a un point de fusion de 32 °C.

2ème stade :

On laisse refroidir le mélange brut obtenu au premier stade jusqu'à 60 °C, puis on ajoute 400 ml d'isopropanol. La température de la solution est ajustée à 40 °C. On ajoute ensuite 12 ml d'une solution aqueuse d'acide chlorhydrique 6 N et on maintient le mélange réactionnel pendant deux heures à 40 °C. Le mélange obtenu est filtré, puis concentré à l'évaporateur rotatif à 80 °C sous pression réduite, afin d'éliminer l'isopropanol.

On laisse refroidir le produit pâteux obtenu qui fige à température ambiante. On obtient 98 g d'une cire jaune clair ayant un point de fusion de 54,2 °C.

L'analyse par chromatographie en phase supercritique et détection par ionisation de flamme montre que le produit a la composition suivante (les pour-cent correspondant à la proportionalité directe de la

surface mesurée des pics) : composé dans lequel :

| | |
|---|---|
| n = 1 | 35,9 % |
| n = 2 | 41,8 % |
| n = 3 | 18,5 % |
| n = 4 | 3,6 % |

EXEMPLE 2 : PREPARATION D'UN COMPOSE DE FORMULE (I)

dans laquelle R = $-CH_2A$, A = -OR', R' = $C_{16}H_{33}$ et $\bar{n}$ = 1,5

Le composé est préparé selon le mode opératoire de l'exemple 1 en utilisant :

- 13,2 g (0,1 mole) d'isopropylidèneglycérol
- 1,17 g (0,010 mole) de tert-butylate de potassium
- 44,7 g (0,15 mole) d'éther d'hexadécyle et de glycidyle
- 225 ml d'isopropanol
- 3,4 ml d'une solution concentrée d'HCl (d = 1,19)

Le produit obtenu est un liquide ambré qui se solidifie en donnant une cire ayant un point de fusion de 55 °C.

L'analyse chromatographique effectuée par la même méthode qu'à l'exemple 1 montre que le produit a la composition suivante : composé dans lequel :

| | |
|---|---|
| n = 1 | 54,2 % |
| n = 2 | 36,1 % |
| n = 3 | 9,6 % |

EXEMPLE 3 : PREPARATION D'UN COMPOSE DE FORMULE (I)

dans laquelle R = $-CH_2A$, A = -OR', R' = $C_{16}H_{33}$ et $\bar{n}$ = 2,5

Le composé est préparé selon le mode opératoire de l'exemple 1 en utilisant :

- 13,2 g (0,1 mole) d'isopropylidèneglycérol
- 1,96 g (0,0175 mole) de tert-butylate de potassium
- 74,5 g (0,25 mole) d'éther d'hexadécyle et de glycidyle
- 200 ml d'isopropanol
- 3,96 ml d'une solution concentrée d'HCl (d = 1,19)

Le produit obtenu est, à température ambiante, une cire de couleur ambrée ayant un point de fusion de 51,6 °C.

L'analyse chromatographique effectuée par la même méthode qu'à l'exemple 1 montre que le produit obtenu a la composition suivante :
composé dans lequel :

| | |
|---|---|
| n = 1 | 22,2 % |
| n = 2 | 43,9 % |
| n = 3 | 27,8 % |
| n = 4 | 5,9 % |

EXEMPLE 4 : PREPARATION D'UN COMPOSE DE FORMULE (I)

dans laquelle R = $-CH_2A$, A = -OR', R' = $C_{16}H_{33}$ et $\bar{n}$ = 3

Le composé est préparé selon le mode opératoire de l'exemple 1 en utilisant :

- 33 g (0,25mole) d'isopropylidèneglycérol
- 5,9 g (0,052 mole) de tert-butylate de potassium
- 223,5 g (0,75 mole) d'éther d'hexadécyle et de glycidyle

- 1 litre d'isopropanol
- 11 ml d'une solution concentrée d'HCl (d = 1,19)

Le produit obtenu est, à température ambiante, une cire brun clair, ayant un point de fusion de 51,9°C.

L'analyse chromatographique effectuée par la même méthode qu'à l'exemple 1 montre que le produit obtenu a la composition suivante :
composé dans lequel :

| | |
|---|---|
| n = 1 | 12 % |
| n = 2 | 37 % |
| n = 3 | 34 % |
| n = 4 | 17 % |

EXEMPLE 5: PREPARATION D'UN COMPOSE DE FORMULE (I)

dans laquelle R = $-CH_2A$, A = -OR', R' = $C_{12}H_{25}$ et $\bar{n}$ = 3

Le composé est préparé selon le mode opératoire de l'exemple 1 en utilisant :
- 13,2 g (0,1 mole) d'isopropylidèneglycérol
- 2,3 g (0,021 mole) de tert-butylate de potassium
- 72,6 g (0,3 mole) d'éther de dodécyle et de glycidyle
- 350 ml d'isopropanol
- 4,25 ml d'une solution concentrée d'acide chlorhydrique (d = 1,18) et 5 ml d'eau

On obtient un produit qui est une huile jaune.

L'analyse chromatographique effectuée par la même méthode qu'à l'exemple 1 montre que le produit a la composition suivante :
composé dans lequel :

| | |
|---|---|
| n = 1 | 20 % |
| n = 2 | 40 % |
| n = 3 | 25 % |
| n = 4 | 11 % |
| n = 5 | 4 % |

EXEMPLE 6: PREPARATION D'UN COMPOSE DE FORMULE (I)

dans laquelle R = $-CH_2A$, A = -OR', R' = $C_8H_{17}$ et $\bar{n}$ = 4

Le composé est préparé selon le mode opératoire de l'exemple 1 en utilisant :
- 13,2 g (0,1 mode) d'isopropylidèneglycérol
- 3,14 g (0,028 mode) de tert-butylate de potassium
- 74,4 g (0,4 mode) d'éther d'octyle et de glycidyle
- 350 ml d'isopropanol
- 4,8 ml d'une solution concentrée d'acide chlorhydrique (d = 1,18) et 5 ml d'eau

Dans le second stade, on chauffe au reflux de l'isopropanol pendant 2 heures.

Le produit obtenu est une huile brune.

L'analyse chromatographique effectuée par la même méthode qu'à l'exemple 1 montre que le produit a la composition suivante :
composé dans lequel :

| | |
|---|---|
| n = 1 | 25,9 % |
| n = 2 | 34,1 % |
| n = 3 | 21,5 % |
| n = 4 | 11,5 % |
| n = 5 | 10,7 % |
| n = 6 | 7,0 % |
| n = 7 à 12 | traces décelables |

EXEMPLE 7 : PREPARATION D'UN COMPOSE DE FORMULE (I)

dans laquelle R = $-CH_2A$, A = -OR', R' = $C_{10}H_{21}$ et $\bar{n}$ = 3

Le composé est préparé selon le mode opératoire de l'exemple 1.

Dans le premier stade, on mélange :
- 13,2 g (0,1 mode) d'isopropylidèneglycérol
- 2,3 g (0,021 mode) de tert-butylate de potassium

On ajoute :
- 68 g (0,32 mole) d'éther de décyle et de glycidyle

A la fin de l'addition, on maintient le chauffage pendant 2 heures à 120 ° C puis pendant 2 heures à 130 ° C.

Dans le second stade, on ajoute :
- 350 ml d'isopropanol
- 4,3 ml d'une solution concentrée d'acide chlorhydrique (d = 1,18) et 5 ml d'eau

On chauffe au reflux de l'isopropanol pendant 3 heures.

Le produit obtenu est une huile brune.

L'analyse chromatographique effectuée par la même méthode qu'à l'exemple 1 montre que le produit a la composition suivante :
composé dans lequel :

| | |
|---|---|
| n = 1 | 34,3 % |
| n = 2 | 42,0 % |
| n = 3 | 17,7 % |
| n = 5 | 5,8 % |

EXEMPLE 8 : PREPARATION D'UN COMPOSE DE FORMULE (I)

dans laquelle R = $-CH_2A$, A = -OR',

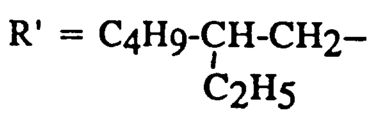

et $\bar{n}$ = 5

Le composé est préparé selon le mode opératoire de l'exemple 1 en utilisant :
- 13,2 g (0,1 mole) d'isopropylidèneglycérol
- 3,9 g (0,035mole) de tert-butylate de potassium
- 93 g (0,5 mole) d'éther de 2-éthylhexyle et de glycidyle
- 400 ml d'isopropanol
- 5,4 ml d'une solution concentrée d'acide chlorhydrique (d = 1,18) et 5 ml d'eau

Dans le second stade, on chauffe au reflux de l'isopropanol pendant deux heures.

Le produit obtenu est une huile brune.

L'analyse chromatographique effectuée par la même méthode qu'à l'exemple 1 montre que le produit a la composition suivante :
composé dans lequel :

| | |
|---|---|
| n = 1 | 10,0% |
| n = 2 | 25,0 % |
| n = 3 | 21,0 % |
| n = 4 | 14,5 % |
| n = 5 | 11,0 % |
| n = 6 | 11,0 % |
| n = 7 | 7,5 % |
| n = 8 à 13 | traces |

EXEMPLE 9 : PREPARATION D'UN COMPOSE DE FORMULE (I)

dans laquelle R = $-CH_2A$, A = -OR', R' = $C_{18}H_{37}$ et $\bar{n}$ = 2

Le composé est préparé selon le mode opératoire de l'exemple 1.

Dans un premier stade, on mélange :

- 13,2 g (0,1 mole) d'isopropylidèneglycérol
- 1,57 g (0,014mole) de tert-butylate de potassium

On ajoute ensuite :

- 65,2 g (0,2 mole) d'éther d'octadécyle et de glycidyle

A la fin de l'addition, on poursuit le chauffage à 150 ° C pendant 4 heures. On ajoute au mélange 0,5 g de tert-butylate de potassium et on poursuit le chauffage à 150 ° C pendant 2 heures.

Dans un second stade, on ajoute :

- 300 ml d'isopropanol
- 4 ml d'une solution concentrée d'acide chlorhydrique (d = 1,18) et 4 ml d'eau

On chauffe au reflux de l'isopropanol pendant deux heures.

Le produit obtenu est une cire beige ayant un point de fusion de 59,1 ° C.

L'analyse chromatographique effectuée par la même méthode qu'à l'exemple 1 montre que le produit a la composition suivante :

composé dans lequel :

| | |
|---|---|
| n = 1 | 38 % |
| n = 2 | 27 % |
| n = 3 | 23 % |
| n = 4 | 9 % |
| n = 5 | 3 % |

EXEMPLE 10 : PREPARATION D'UN COMPOSE DE FORMULE (I)

dans laquelle R = $-CH_2A$, A = -OR', R' = $C_3-(CH_2)_7-CH = CH-(CH_2)_8-$, $\bar{n}$ = 2

Le composé est préparé selon le mode opératoire de l'exemple 1.

Dans un premier stade, on mélange :

- 6,6 g (0,05 mole) d'isopropylidèneglycérol
- 1,57 g (0,014mole) de tert-butylate de potassium

On ajoute ensuite :

- 32,4g (0,1 mole) d'éther d'oléyle et de glycidyle

A la fin de l'addition, le mélange réactionnel est chauffé à 150 ° C pendant 5 heures.

Dans un second stade, on ajoute :

- 150 ml d'isopropanol
- 2,5 ml d'une solution concentrée d'acide chlorhydrique (d = 1,18) et 2,5 ml d'eau

On chauffe à 80 ° C pendant 4 heures.

Le produit obtenu est une huile brune.

Le spectre $RMN^{13}$ C est en accord avec la formule.

L'analyse chromatographique effectuée par la même méthode qu'à l'exemple 1 montre que le produit a la composition suivante :

composé dans lequel :

| | |
|---|---|
| n = 1 | 27 % |
| n = 2 | 37 % |
| n = 3 | 26 % |
| n = 4 | 8 % |
| n = 5 | 2 % |

<u>EXEMPLE 11 : PREPARATION D'UN COMPOSE DE FORMULE (I)</u>

dans laquelle R = $-CH_2A$, A = -OR', R' = isostéaryle (*) $\bar{n}$ = 2

Le composé est préparé selon le mode opératoire de l'exemple 1.

Dans le premier stade, on mélange :

- 13,2 g (0,1 mole) d'isopropylidèneglycérol
- 3,0 g (0,027mole) de tert-butylate de potassium

On ajoute ensuite :

- 70 g (0,2 mole) d'éther d'isostéaryle et de glycidyle

Après l'addition, on chauffe pendant 5 heures à 150 °C.

Dans le second stade, on ajoute :

- 350 ml d'isopropanol et
- 4,2 ml d'une solution concentrée d'HCl (d = 1,18)

On chauffe pendant 4 heures à 60 °C.

Le produit obtenu est une huile brune.

L'analyse chromatographique effectuée par la même méthode qu'à l'exemple 1 montre que le produit a la composition suivante :

composé dans lequel :

| | |
|---|---|
| n = 1 | 27 % |
| n = 2 | 44 % |
| n = 3 | 21 % |
| n = 4 | 6 % |
| n = 5 | 2 % |

<u>EXEMPLE 12 : PREPARATION D'UN COMPOSE DE FORMULE (I)</u>

dans laquelle R = $-CH_2A$,
-A = -OR',

$$R' = CH_3\text{-}(CH_2)_9\text{-}CH\text{-}CH_2\text{-}$$
$$C_8H_{17}$$

et n = 2

Le composé est préparé selon le mode opératoire de l'exemple 1.

Dans le premier stade, on mélange :

- 13,2 g (0,1 mole) d'isopropylidèneglycérol
- 2,25 g (0,02mole) de tert-butylate de potassium

On ajoute ensuite :

- 70,8 g (0,2 mole) d'éther de 2-octyldodécyle et de glycidyle

A la fin de l'addition, on poursuit le chauffage à 150 °C pendant 2 heures.

Dans le second stade, on mélange :

- 350 ml d'isopropanol et

(*) le radical isostéaryle est un mélange de radicaux alkyle isomères en $C_{18}$.

- 4,2 ml d'une solution concentrée d'HCl (d = 1,18)

On chauffe à 80 ° C pendant 4 heures.

Le produit obtenu est une huile brune.

L'analyse chromatographique effectuée par la même méthode qu'à l'exemple 1 montre que le produit a la composition suivante :

composé dans lequel :

| | |
|---|---|
| n = 1 | 22 % |
| n = 2 | 40 % |
| n = 3 | 29 % |
| n = 4 | 9 % |

EXEMPLE 13 : PREPARATION D'UN COMPOSE DE FORMULE (I)

dans laquelle R = $-CH_2A$,

- A = -OR',

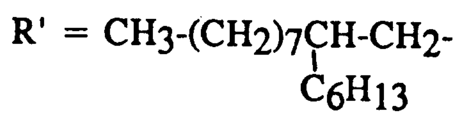

et $\bar{n}$ = 2

Le composé est préparé selon le mode opératoire de l'exemple 1.

Dans le premier stade, on mélange :

- 13,2 g (0,1 mole) d'isopropylidèneglycérol et
- 4,4 g (0,04mole) de tert-butylate de potassium

On ajoute ensuite :

- 59,6 g (0,2 mole) d'éther de 2-hexyldécyle et de glycidyle

A la fin de l'addition, on poursuit le chauffage à 150 ° C pendant 6 heures.

Dans le second stade, on ajoute :

- 350 ml d'isopropanol
- 7,5 ml d'une solution d'acide chlorhydrique concentrée (d = 1,18)

On chauffe à 80 ° C pendant 2 heures.

Le produit obtenu est une huile brune.

L'analyse chromatographique effectuée par la même méthode qu'à l'exemple 1 montre que le produit a la composition suivante :

composé dans lequel :

| | |
|---|---|
| n = 1 | 19,0 % |
| n = 2 | 23,0 % |
| n = 3 | 25,0 % |
| n = 4 | 15,0 % |
| n = 5 | 9,5 % |
| n = 6 | 4,5 % |
| n = 7 | 3,0 % |
| n = 8 | 1,0 % |

EXEMPLE 14 : PREPARATION D'UN COMPOSE DE FORMULE (I)

dans laquelle R = $-CH_2A$, A = -OR',

R' = 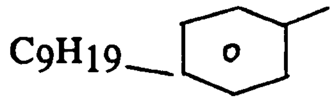

et $\bar{n}$ = 2

Le composé est préparé selon le mode opératoire de l'exemple 1.

Dans le premier stade, on mélange :

- 13,2 g (0,1 mole) d'isopropylidèneglycérol et
- 3,3 g (0,03 mole) de tert-butylate de potassium

On ajoute ensuite :

- 56 g (0,2 mole) d'éther de nonylphényle et de glycidyle

A la fin de l'addition, on poursuit le chauffage à 150 °C pendant 2 heures.

Dans le second stade, on ajoute :

- 300 ml d'isopropanol
- 5 ml d'une solution d'acide chlorhydrique concentrée (d = 1,19)

On chauffe au reflux de l'isopropanol pendant 2 heures.

Le produit obtenu est une huile brune très visqueuse.

L'analyse chromatographique effectuée par la même méthode qu'à l'exemple 1 montre que le produit a la composition suivante :

composé dans lequel :

| | |
|---|---|
| n = 1 | 15 % |
| n = 2 | 32 % |
| n = 3 | 31 % |
| n = 4 | 16 % |
| n = 5 | 6 % |
| n = 6 | traces |

EXEMPLE 15 : PREPARATION D'UN COMPOSE DE FORMULE (I)

dans laquelle R = $C_{10}H_{21}$ ; $\bar{n}$ = 3

Le composé est préparé selon le mode opératoire de l'exemple 1.

Dans le premier stade, on mélange :

- 13,2 g (0,1 mole) d'isopropylidèneglycérol et
- 6,74 g (0,06 mole) de tert-butylate de potassium

On ajoute ensuite :

- 55,2 g (0,3 mole) de 1,2-époxydodécane

Après addition, le chauffage est poursuivi à 180 °C pendant 6 heures.

Dans le second stade, on ajoute :

- 300 ml d'isopropanol et
- 15 ml d'une solution d'acide chlorhydrique 6 N

On chauffe au reflux du solvant pendant 3 heures.

L'analyse chromatographique effectuée par la même méthode qu'à l'exemple 1 montre que le produit a la composition suivante :

composé dans lequel :

| | |
|---|---|
| n = 1 | 22 % |
| n = 2 | 45 % |
| n = 3 | 25 % |
| n = 4 | 8 % |

EXEMPLE 16 : PREPARATION D'UN COMPOSE DE FORMULE (I)

dans laquelle R = $C_{12}H_{25}$ et $\overline{n}$ = 3

Le composé est préparé selon le mode opératoire de l'exemple 1.

Dans le premier stade, on mélange :

- 13,2 g (0,1 mole) d'isopropylidèneglycérol et
- 2,36 g (0,021 mole) de tert-butylate de potassium

On ajoute ensuite :

- 63,6 g (0,3 mole) de 1,2-époxytétradécane

Après addition, on poursuit le chauffage pendant 2 heures à 140°C. On ajoute alors 2,36 g de tert-butylate de potassium et on poursuit le chauffage pendant 4 heures à 140°C.

Dans le second stade, on ajoute :

- 300 ml d'isopropanol et
- 15 ml d'une solution d'acide chlorhydrique 6 N

On chauffe au reflux du solvant pendant 3 heures.

On obtient 71,5 g d'un produit cireux jaune.

L'analyse chromatographique effectuée par la même méthode qu'à l'exemple 1 montre que le produit a la composition suivante :

composé dans lequel :

| | |
|---|---|
| n = 1 | 17 % |
| n = 2 | 40 % |
| n = 3 | 23 % |
| n = 4 | 9 % |
| n = 5 | 4 % |
| n = 6 | 4 % |
| n = 7 | 3 % |

EXEMPLE 17 : PREPARATION D'UN COMPOSE DE FORMULE (I)

dans laquelle R = $C_{14}H_{29}$ et $\overline{n}$ = 2

Le composé est préparé selon le mode opératoire de l'exemple 1.

Dans le premier stade, on mélange :

- 33 g (0,25 mole) d'isopropylidèneglycérol et
- 3,92 g (0,035 mole) de tert-butylate de potassium

On ajoute ensuite :

- 132 g (0,50 mole) de 1,2-époxyhexadécane

Après addition, on chauffe à 140°C pendant 2 heures.

Dans le second stade, on ajoute :

- 660 ml d'isopropanol et
- 7,35 ml d'une solution d'acide chlorhydrique (d = 1,18)

On maintient le mélange pendant 4 heures à 60°C.

On obtient 127,5 g d'un produit brun clair.

L'analyse chromatographique effectuée par la même méthode qu'à l'exemple 1 montre que le produit a la composition suivante :

composé dans lequel :

| | |
|---|---|
| n = 1 | 37 % |
| n = 2 | 40 % |
| n = 3 | 17 % |
| n = 4 | 6 % |

EXEMPLE 18 : PREPARATION D'UN COMPOSE DE FORMULE (I)

dans laquelle R = $C_{14}H_{29}$ et $\overline{n}$ = 3

Le composé est préparé selon le mode opératoire de l'exemple 1.

Dans le premier stade, on mélange :

- 33 g (0,25 mole) d'isopropylidèneglycérol et
- 5,9 g (0,052 mole) de tert-butylate de potassium

On ajoute ensuite :

- 180 g (0,75 mole) de 1,2-époxyhexadécane

Après addition, on poursuit le chauffage à 140 °C pendant 2 heures.

Dans le second stade, on ajoute :

- 1 litre d'isopropanol et
- 11 ml d'acide chlorhydrique concentré

On chauffe pendant 4 heures à 60 °C.

On obtient 174 g d'un produit brun clair.

L'analyse chromatographique effectuée par la même méthode qu'à l'exemple 1 montre que le produit a la composition suivante :

composé dans lequel :

| | |
|---|---|
| n = 1 | 20 % |
| n = 2 | 44 % |
| n = 3 | 24 % |
| n = 4 | 6 % |
| n = 5 | 6 % |

EXEMPLE 19 : PREPARATION D'UN COMPOSE DE FORMULE (I)

dans laquelle R = $C_{16}H_{33}$ et $\overline{n}$ = 2

Le composé est préparé selon le mode opératoire de l'exemple 1.

Dans le premier stade, on mélange :

- 9,9 g (0,075 mole) d'isopropylidèneglycérol et - 1,77 g (0,016 mole) de tert-butyle de potassium

On ajoute ensuite :

- 40,2 g (0,15 mole) de 1,2-époxyoctodécane

A la fin de l'addition, on poursuit le chauffage à 180 °C pendant 4 heures.

Dans le second stade, on ajoute :

- 300 ml d'isopropanol et
- 3,71 inl d'acide chlorhydrique concentré

On chauffe pendant 2 heures à 80 °C.

On obtient 44 g d'un produit brun.

L'analyse chromatographique effectuée par la même méthode qu'à l'exemple 1 montre que le produit a la composition suivante :

composé dans lequel :

| | |
|---|---|
| n = 1 | 48 % |
| n = 2 | 38 % |
| n = 3 | 8 % |
| n = 4 | 6 % |

EXEMPLE 20 : PREPARATION D'UN COMPOSE DE FORMULE (I)

dans laquelle R est un mélange de radicaux alkyle en $C_{22}$ - $C_{26}$ et $\bar{n}$ = 2

Le composé est préparé selon le mode opératoire de l'exemple 1.

Dans le premier stade, on mélange :

- 13,2 g (0,1 mole) d'isopropylidèneglycérol et
- 1,57 g (0,014 mole) de tert-butylate de potassium

On ajoute ensuite :

- 85,4 g (0,2 mole) de "VIKOLOX 24-28" (qui est un mélange d'époxydes de formule (III) dans laquelle R est un mélange de radicaux en $C_{22}$ à $C_{26}$ commercialisé par la Société VIKING). On maintient le chauffage pendant 4 heures à 140 °C.

On ajoute à nouveau 1,57 g de tert-butylate de potassium et on poursuit le chauffage à 140 °C pendant 4 heures.

Dans le second stade, on ajoute :

- 300 ml d'isopropanol et
- 6 ml d'acide chlorhydrique concentré

On chauffe pendant 4 heures à reflux du solvant.

On obtient 63 g d'un produit beige qui fige à la température ambiante et dont le point de fusion est de 75-80 °C.

EXEMPLE 21 : PREPARATION DES COMPOSES DE FORMULE I

dans lesquels R = $-CH_2A$, A = OR', R' = $C_{16}$ - $H_{33}$ et n = 1 (non compris dans l'invention) et n = 2.

L'éther d'hexadécyle et de glycidyle (50 g ; 0,17 mole) est solubilisé dans 44,25 g (3,35 moles) d'isopropylidèneglycérol. D'autre part, 44,25 g (3,35 moles) d'isopropylidèneglycérol sont introduits dans un réacteur avec 1,5 g (0,013 mole) et de tert-butylate de potassium. On chauffe le milieu réactionnel à 140 °C et à cette température, on introduit la solution d'éther d'hexadécyle et de glycidyle dans l'isopropylidèneglycérol pendant 2 heures. On chauffe pendant encore 1 heure à 140 °C après l'addition. Après refroidissement, on ajoute 120 ml d'une solution de NaCl (10 g dans 150 ml d'eau). On décante et on élimine la phase aqueuse ; à la phase organique, on ajoute 350 ml d'hexane. On effectue une nouvelle décantation. On élimine une petite phase brune. La phase organique est ensuite séchée sur sulfate de sodium. On élimine le solvant sous une pression réduite de 53 x $10^2$ Pascals, puis l'isopropylidèneglycérol sous une pression réduite de 1,3 x $10^2$ Pascals.

On recueille le produit final avec un rendement de 94 %.

Ce produit est soumis à une distillation à 150 °C.

On isole le compose dans lequel n = 1 au niveau des produits légers avec un rendement de 90 % ; c'est une huile limpide quasi incolore, qui fige à température ambiante.

Les produits lourds contiennent essentiellement le composé dans lequel n = 2. Ils sont mis en solution dans le méthanol au reflux puis additionnés d'acide chlorhydrique concentré (pour 589 g de résidu, 53 ml HCl 5 N et 250 ml de méthanol). Le mélange est maintenu au reflux pendant 3 heures ; puis on laisse refroidir pendant une nuit.

Le précipité est recueilli par filtration puis dissous dans l'acétate d'éthyle et filtré à chaud pour éliminer l'insoluble (250 ml d'acétate d'éthyle pour 50 g de produit brut). On laisse recristalliser pendant une nuit à 8 °C puis on filtre sur verre fritté. Le gâteau essoré est recristallisé une seconde fois dans 200 ml de solvant.

Après séchage, on obtient 24,7 g d'une poudre beige claire ayant pour point de fusion 54,1 °C.

L'analyse chromatographique en phase super-critique et détection par ionisation de flamme indique la présence résiduelle de 7 % en poids de composé dans lequel n = 1 et 6,5 % de composé dans lequel n = 3, le reste étant constitué par le composé dans lequel n = 2 (les pour-cent correspondant à la proportionalité de la surface mesurée des pics).

EXEMPLES 22 A 29 : FORMULATIONS

EXEMPLE 22 (comparatif)

On a préparé des dispersions de vésicules à partir de composés amphiphiles non-ioniques de formule (I) selon l'invention dans laquelle n a des valeurs différentes, R est $C_{14}H_{29}$ ou R est $-CH_2A$, A étant -OR', R' étant $C_{16}H_{33}$ et, à titre comparatif, avec trois composés amphiphiles non-ioniques ne faisant pas partie de

l'invention, de formule :

$$C_{16}H_{33}-O-[C_2H_3O-(CH_2OH)]_3-H \quad (F1)$$

$-C_2H_3O(CH_2OH)-$ représentant les structures suivantes prises en mélange ou séparément :

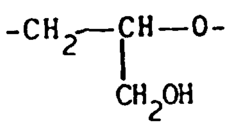 ; 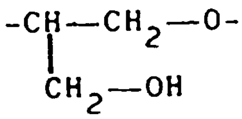

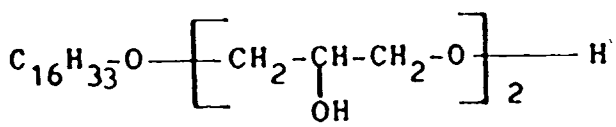 (F2)

et 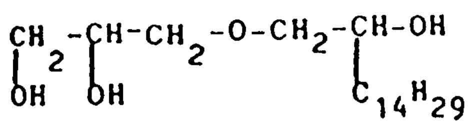 (F3)

comportant une seule chaîne lipophile.

1) PREPARATION DES PHASES VESICULAIRES

Dans un bécher en verre, on pèse les produits suivants :

| | |
|---|---|
| - amphiphile non-ionique | 9,00 g |
| - cholestérol | 5,25 g |
| - dicétylphosphate | 0,75 g |

On réalise le mélange de ces trois produits par fusion à la température de 100°C sous atmosphère d'azote. Puis, on ramène la température du mélange fondu à 90°C.

On ajoute 30 g d'eau déminéralisée et on homogénéise le mélange obtenu à la température de 90°C.

i) A une première fraction du produit obtenu correspondant à 15 g de lipides, on ajoute de l'eau déminéralisée et on disperse à 70°C à l'aide d'un ultradisperseur de type "VIRTIS" pendant 4 minutes à la vitesse de 40000 t/min, de façon à obtenir une dispersion (c) de vésicules à 15 % en poids de lipides ; les vésicules ne sont pas chargées en produits actifs. On mesure ensuite la viscosité de la dispersion (c) de vésicules au moins 18 heures après sa fabrication.

ii) A une seconde fraction du produit obtenu correspondant à 15 g de lipides, on ajoute 5,57 g de glycérine et 24 g d'eau déminéralisée.

A la température de 70°C, on homogénéise le mélange à l'aide d'un ultradisperseur du type "VIRTIS" pendant 4 minutes à la vitesse de 40000 t/min.

On ajoute alors :

- 13,94 g d'une solution aqueuse à 20 % en poids de poly -alanine ;
- 9,3 g d'une solution aqueuse à 1 % en poids de lactate de monométhyl trisilanol vendu par la Société Jean D'AVEZ sous la dénomination commerciale "LASILIUM" ;
- 0,56 g de stabilisateur constitué de diazolidinyl-urée vendue par la Société SUTTON sous la dénomination commerciale "GERMALL II" ;
- 0,56 g de stabilisateur constitué par un mélange de méthylchloroisothiazolinone et de méthylisothiazolinone vendu par la Société ROHM et HAAS sous la dénomination commerciale "KATHON CG" dissous dans 1 g d'eau déminéralisée.

A la température de 40°C, on homogénéise le mélange à l'aide d'un ultradisperseur "VIRTIS" pendant 2 minutes à la vitesse de 40000 t/min.

On constate qu'avec le produit de formule ($F_3$) ne faisant pas partie de l'invention, il n'est pas possible d'obtenir des vésicules dans les conditions utilisées.

Avec les autres composés, on obtient une dispersion (a) de vésicules chargées en produits actifs, de tailles inférieures à 0,3 micron, dont on mesure la viscosité au moins 18 heures après la fabrication.

2) ADDITION D'UNE PHASE GRASSE POUR LA PREPARATION D'UNE CREME

On ajoute à la dispersion (a) de vésicules chargées en produits actifs :

- 27,9 g d'huile de macadamia ; et
- 18,6 g d'huile de silicone volatile.

A la température de 30 ° C, on homogénéise le tout à l'aide d'un ultradisperseur du type "VIRTIS" pendant 4 minutes à la vitesse de 40000 t/min. On obtient ainsi une crème (b) blanche épaisse, dont on mesure la viscosité au moins 18 heures après la fabrication.

Les résultats des différentes mesures de viscosité sont donnés dans le tableau I ci-après.

TABLEAU I

| AMPHIPHILE NON-IONIQUE | NOMBRE DE CHAINES LIPOPHILES | VISCOSITE DE LA DISPERSION (a) (Pascals.secondes) | VISCOSITE DE LA DISPERSION (c) (Pascals.secondes) | VISCOSITE DE LA CREME (b) (Pascals.secondes) |
|---|---|---|---|---|
| $C_{16}H_{33}O - [C_2H_3O-(CH_2OH)]_3 - H$ * | 1 | 0,02 | - | 0,11 |
| $C_{16}H_{33}O - [CH_2-CH(OH)-CH_2-O]_2 - H$ * | 1 | 0,02 | 2,4 | 0,15 |
| $HO-[CH-CH_2]_n-OCH_2-CH-CH_2OH$<br>$\quad\ CH_2 \qquad\qquad\qquad OH$<br>$\quad\ O-C_{16}H_{33}$ | $\overline{1,5}$ (ex 2)<br>$\overline{2}$ (ex 1)<br>2 (ex 21)<br>$\overline{2,5}$ (ex 3)<br>$\overline{3}$ (ex 4) | 0,2<br>1<br>0,3<br>0,4<br>0,13 | -<br>2<br>-<br>-<br>- | 0,60<br>0,66<br>0,94<br>2,33<br>3,75 |
| $HO-[CH-CH_2]_n O-CH_2-CH-CH_2OH$<br>$\quad C_{14}H_{32} \qquad\qquad OH$ | $\overline{2}$ (ex 17)<br>$\overline{3}$ (ex 18) | 0,10<br>0,70 | -<br>- | 0,30<br>0,66 |
| * Ne fait pas partie de l'invention | | | | |

Sur ce tableau, on constate que les dispersions de vésicules chargées en produits actifs préparées à partir d'amphiphiles à plusieurs chaînes grasses selon l'invention présentent une viscosité plus élevée que celles obtenues à partir de vésicules comportant des amphiphiles à une seule chaîne ; que la viscosité des

mêmes vésicules non chargées en actif en solution dans l'eau est du même ordre de grandeur ; et que, par contre, la viscosité en présence d'une phase grasse est nettement plus élevée. Les composés amphiphiles à plusieurs chaînes lipophiles limitent donc dans les crèmes l'effet fluidifiant des actifs cosmétiques utilisés.

EXEMPLE 23 - Crème de jour anti-âge pour le visage

1ère phase : Préparation de la dispersion de vésicules

Dans un bécher de verre, on pèse les produits suivants :

| | |
|---|---|
| - lipide amphiphile non-ionique de l'exemple 17<br>- cholestérol<br>- dicétylphosphate | 4,8 g<br>2,8 g<br>0,4 g |

On réalise le mélange des lipides par fusion à la température de 100°C sous atmosphère d'azote. Puis, on ramène la température du mélange fondu à 90°C. On ajoute 27,0 g d'eau déminéralisée et on homogénéise le mélange obtenu à la température de 90°C.

On ajoute alors les composés suivants :

| | |
|---|---|
| - glycérine | 3,0 g |
| - L-hydroxyproline | 1,0 g |
| - D-panthénol | 1,5 g |
| - guanosine | 0,01 g |
| - conservateur q.s. | |
| - solution aqueuse à 20 % de poly-β-alanine préparée selon le brevet FR-A-2508795 | 7,5 g |
| - polyphosphonate commercialisé sous la dénomination "DEQUEST 2046" par la société MONSANTO CHEMICAL | 0,8 g |
| - hydrolysat lactique commercialisé sous la dénomination "LACTOLAN LS" par la société LABORATOIRES SEROBIOLOGIQUES | 5,0 g |
| - L-serine | 0,2 g |

A la température de 70°C, on homogénéise le mélange à l'aide d'un ultradisperseur de type "VIRTIS" pendant 4 minutes à la vitesse de 40000 t/min. On obtient ainsi une dispersion de vésicules.

2ème phase : Formulation de la crème

A la dispersion obtenue, on ajoute les substances suivantes :

| | |
|---|---|
| - parahydroxybenzoate de propyle | 0,05 g |
| - huile de macadamia | 7,0 g |
| - concentrats naturels de tocophérols commercialisés par la société PROCHIMEX | 4,0 g |
| - 2-éthylhexyl méthoxy cinnamate commercialisé sous la dénomination de "PARSOL MCX" par la société GIVAUDAN | 0,5 g |
| - 2-hydroxy 4-méthoxy benzophénone commercialisée sous la dénomination "UVINUL M 40" par la société BASF | 0,5 g |
| - huile de silicone volatile | 7,5 g |
| - glycérides de vitamine F commercialisés par la société DUBOIS | 3,0 g |
| - solution aqueuse de superoxyde dismutase vendue par la société PENTAPHARM à 5000 unités par ml | 1,0 g |
| - parfum | 0,2 g |
| - acide polyacrylique réticulé commercialisé sous la dénomination "CARBOPOL 940" par la société GOODRICH | 0,5 g |
| - parahydroxybenzoate de méthyle | 0,2 g |
| - L-lysine monohydrate | 1,0 g |
| - eau q.s.p | 100,0 g |

A la température de 40 °C, on homogénéise le mélange à l'aide de l'ultradisperseur "VIRTIS" pendant 2 minutes à la vitesse de 40000 t/min. On obtient ainsi une crème ayant une viscosité de 6 pascals.secondes et un pH de 6,5.

Le lipide de l'exemple 17 peut être remplacé par ceux des exemples 1 ou 18.

EXEMPLE 24 : CREME DE JOUR HYDRATANTE POUR LE VISAGE

On prépare la crème comme dans l'exemple 23.

Dans la 1ère phase, on prépare une dispersion de vésicules ayant la composition suivante :

| | |
|---|---|
| - lipide amphiphile non-ionique de l'exemple 1 | 3,6 g |
| - cholestérol | 2,1 g |
| - dicetyl phosphate | 0,3 g |
| - acétate d'alpha tocophérol | 0,3 g |
| - glycérine | 5,0 g |
| - eau | 35,0 g |
| - stabilisateur constitué par un mélange de méthylchloroisothiazolinone et de méthylisothiazolinone vendu par la Société ROHM et HAAS sous la dénomination "KATHON CG" | 0,05 g |
| - stabilisateur constitué de diazolidinyl-urée vendu par la société SUTTON sous la dénomination commerciale "GERMALL II" | 0,3 g |
| - acide citrique | 0,02 g |
| - eau | 1,0 g |

Dans la 2ème phase, on ajoute à la dispersion les composés suivants :

| | |
|---|---|
| - huile de macadamia | 10,0 g |
| - octyl dodecanol vendu sous la dénomination "EUTANOL G" par la société HENKEL | 5,0 g |
| - huile de silicone volatile | 5,0 g |
| - 2-éthylhexyl méthoxy cinnamate commercialisé sous la dénomination de "PARSOL MCX" par la Société GIVAUDAN | 0,5 g |
| - 2-hydroxy 4-méthoxy benzophénone commercialisée sous la dénomination "UVINUL M 40" par la Société BASF | 0,5 g |
| - paraoxybenzoate de propyle | 0,05 g |
| - acide polyacrylique réticulé commercialisé sous la dénomination "CARBOPOL 940" par la société GOODRICH | 0,5 g |
| - paraoxybenzoate de méthyle | 0,2 g |
| - triéthanolamine | 0,48 g |
| - eau | qsp 100 g |

EXEMPLE 25

On prépare un bâton de rouge à lèvres ayant la composition suivante :

| | |
|---|---|
| - ozokérite | 12,0 g |
| - cire microcristalline | 4,0 g |
| - cire de Candellila | 6,0 g |
| - composé de l'exemple 9 | 5,0 g |
| - huile de Jojoba | 10,0 g |
| - huile de ricin | 20,0 g |
| - lanoline | 15,0 g |
| - lanoline acétylée | 9,0 g |
| - huile de vaseline | 9,0 g |
| - D and C Red 7 calcium lake | 4,2 g |
| - D and C Red 7 barium lake | 2,3 g |
| - FDC Yellow 5 | 0,8 g |
| - bioxyde de titane | 2,5 g |
| - butylhydroxytoluène | 0,2 g |
| - parfum q.s. | |

On mélange les huiles à une température de 50 à 60°C. Les pigments et laques organiques sont broyés dans la phase huileuse.

On ajoute alors les cires fondues puis le parfum.

La composition est alors coulée dans un moule.

EXEMPLE 26

On prépare une émulsion eau-dans-l'huile de la façon suivante :

On mélange par chauffage à 80°C :

- 25 g d'huile de paraffine
- 2 g de monooléate de sorbitan polyoxyéthyléné à 20 moles d'O.E. vendu sous la dénomination "TWEEN 80" par la société ICI
- 2 g du composé de l'exemple 11.

On ajoute lentement, sous agitation, 71 g d'eau distillée chauffée à 70°C. On laisse refroidir à température ambiante sous vive agitation pendant 10 minutes.

On obtient une émulsion eau-dans-l'huile fine, homogène et stable.

EXEMPLE 27 : BAUME APRES RASAGE

Dans un premier stade, on prépare une dispersion vésiculaire de la façon suivante :
On réalise par fusion à la température de 90 - 95 °C, le mélange des produits suivants :

| | |
|---|---|
| - lipide de l'exemple 17 | 1,9 g |
| - stéaroylglutamate mono sodique vendue sous la dénomination d'"ACYL GLUTAMATE HS 11" par la société AJINOMOTO | 0,2 g |
| - cholestérol | 1,9 g |

On ajoute 8 g d'eau déminéralisée et on homogénéise le mélange obtenu.
Puis on ajoute, à température ambiante, les composés suivants :

| | |
|---|---|
| - parahydroxybenzoate de méthyle | 0,1 g |
| - diméthylol-1,3 diméthyl-5,5 hydantoïne vendu sous la dénomination de "GLYDANT" par la Société GLYCO | 0,055 g MA |
| - eau déminéralisée | 12,0 g |

On agite pour obtenir une dispersion de vésicules.
On ajoute les substances suivantes pour obtenir le baume :

| | |
|---|---|
| - lanoline liquide protégée vendue sous la dénomination de "STELLANOL" par la société STELLA | 1,0 g |
| - mélange de polydiméthylsiloxane, α ω-dihydroxylé/cyclotétradiméthyl siloxane / cyclopentadiméthyl siloxane (14/48,2/37,8) vendu sous la dénomination "DOW CORNING QCF2-1671" par la société DOW CORNING | 5,0 g |
| - parfum | 0,5 g |
| - polymère carboxyvinylique vendu sous la dénomination de "CARBOPOL 940" par la société GOODRICH | 0,2 g |
| - triéthanolamine qs pH 6,5 | |
| - eau déminéralisée qsp | 100,0 g |

On constate que, dans le baume, les vésicules restent stables.

EXEMPLE 28 : CREME AUTO-BRONZANTE

On prépare, comme dans l'exemple 27, une crème contenant des vésicules de lipides non-ioniques ayant la composition suivante :

| | |
|---|---|
| - lipide de l'exemple 17 | 3,8 g |
| - cholestérol | 3,8 g |
| - stéaroylglutamate mono sodique vendu sous la dénomination d'"ACYL GLUTAMATE HS 11" par la Société AJINOMOTO | 0,4 g |
| - eau déminéralisée | 16,0 g |
| - glycérine | 2,0 g |
| - dihydroxyacétone | 5,0 g |
| - conservateurs qs | |
| - eau déminéralisée | 24,0 g |
| - huile de vaseline vendue sous la dénomination de "SIDEPALINE BC 15" par la Société GEERAERT MATTHYS | 15,0 g |
| - parfum qs | |
| - hydroxyéthylcellulose hydrophobe vendue sous la dénomination de "NATROSOL PLUS GRADE 330 CS" par la société AQUALON | 0,5 g |
| - eau déminéralisée qsp | 100,0 g |

On constate que les vésicules restent stables dans la crème.
Le lipide de l'exemple 17 pourrait être remplacé par celui de l'exemple 1.

EXEMPLE 29 : CREME SOLAIRE

On prépare comme dans l'exemple 27, une crème contenant des vésicules de lipides non-ioniques.

| | |
|---|---|
| - lipide de l'exemple 1 | 4,8 g |
| - cholestérol | 2,8 g |
| - stéaroylglutamate monosodique vendu sous la dénomination d'"ACYL GLUTAMATE HS 11" par la Société AJINOMOTO | 0,4 g |
| - eau déminéralisée | 16,0 g |
| - conservateurs qs | |
| - eau déminéralisée | 24,0 g |
| - huile de vaseline vendue sous la dénomination de "SIDEPALINE BC 15" par la société GEERAERT MATTHYS | 15,0 g |
| - paraméthoxycinnamate de 2-éthylhexyle vendu sous la dénomination "PARSOL MCX" par la Société GIVAUDAN | 4,0 g |
| - 2-hydroxy 4-méthoxybenzophénone vendu sous la dénomination "UVINUL M40" par la Société BASF | 2,0 g |
| - parfum qs | |
| - polymère carboxyvinylique vendu sous la dénomination de "CARBOPOL 940" par la Société GOODRICH | 0,4 g |
| - eau déminéralisée qsp | 100,0 g |

On constate que, dans la crème, les vésicules restent stables.

**Revendications**

**Revendications pour les Etats contractants suivants : AT, BE, CH, DE, DK, FR, GB, IT, LI, LU, NL, SE**

**1.** Composés amphiphiles non-ioniques dérivés du glycérol, caractérisés par le fait qu'ils ont la formule (I):

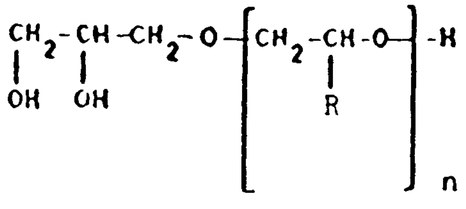

(I)

formule dans laquelle :

- R représente un radical pris dans le groupe formé par les radicaux alkyle ou alcényle, linéaires ou ramifiés, en $C_4$ - $C_{28}$ et leurs mélanges ou représente un groupement $-CH_2A$ dans lequel A représente -OR' -SR' ou

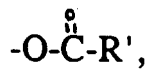

R' représentant un radical hydrocarboné saturé ou insaturé et

- n représente une valeur statistique moyenne $\bar{n}$ supérieure à 1 et au plus égale à 6 et, lorsque R est $-CH_2A$, représente également une valeur égale à 2.

**2.** Composé selon la revendication 1, caractérisé par le fait que le radical hydrocarboné R' est un radical alkyle linéaire en $C_8$ - $C_{22}$, un radical alkyle ramifié en $C_8$ - $C_{36}$, un radical alcényle en $C_{18}$ ou un radical alkylaryle à chaîne alkyle linéaire ou ramifié en $C_8$ - $C_{16}$.

**3.** Composé selon la revendication 2, caractérisé par le fait que dans le radical alkylaryle le groupe aryle est un radical phényle.

**4.** Composé selon la revendication 2, caractérisé par le fait que le radical alcényle est un radical octadécène-9 yle ou octadécanediène 9,12 yle.

**5.** Composé selon la revendication 1, caractérisé par le fait que le radical R représente un radical alkyle linéaire en $C_{14}$ - $C_{18}$ ou un groupement $-CH_2A$ dans lequel A est OR' R' représentant un radical alkyle linéaire en $C_{10}$ - $C_{18}$ et n représente une valeur statistique moyenne $\bar{n}$ supérieure à 1 et au plus égale à 3 et, lorsque R représente $-CH_2A$ est également égal à 2.

**6.** Procédé de préparation de composés de formule I selon l'une des revendications 1 à 5 par un procédé en deux stades avec formation de produit intermédiaire, caractérisé par le fait que :

- dans un premier stade, on fait réagir, en présence d'un catalyseur basique, de l'isopropylidèneglycérol de formule (IV) avec un époxyde de formule (III) dans lequel R a la même signification que dans la formule (I), pour obtenir un (des) produit(s) intermédiaire(s) de formule (II) selon le schéma de réaction suivant :

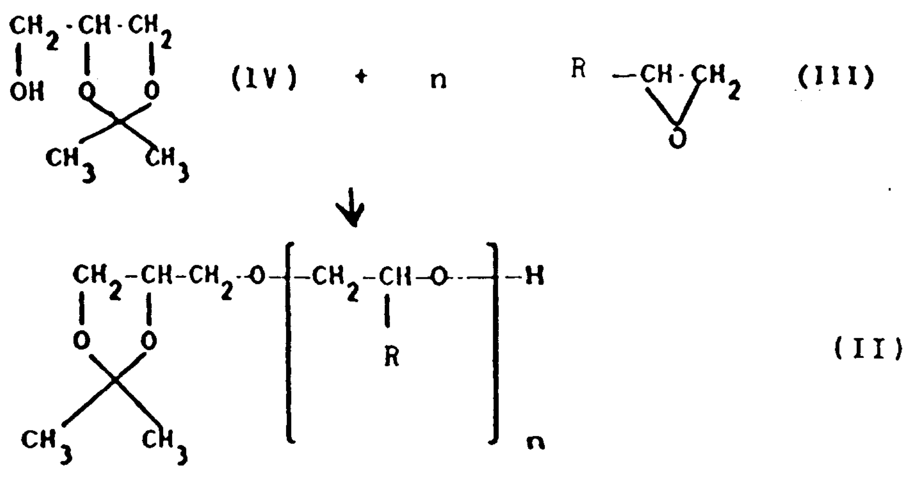

(IV) + n (III)

(II)

R et n ayant la même signification que dans la formule (I) et

- dans un second stade, on hydrolyse le (les) produit(s) intermédiaire(s) de formule (II) obtenu(s) et l'on sépare le (les) composé(s) de formule (I) du mélange réactionnel.

**7.** Procédé selon la revendication 6, caractérisé par le fait que le catalyseur basique utilisé au premier stade est choisi dans le groupe formé par les métaux alcalins, les hydrures de métaux alcalins, les hydroxydes alcalins, les alcoolates alcalins et les fluorures de métaux alcalins.

**8.** Procédé selon la revendication 7, caractérisé par le fait que le catalyseur basique est le tert-butylate de potassium.

**9.** Procédé selon l'une des revendications 6 à 8, caractérisé par le fait que la quantité de catalyseur basique utilisée est comprise entre 4 et 40 % en moles par rapport à l'isopropylidèneglycérol de formule (IV).

**10.** Procédé selon l'une des revendications 6 à 9, caractérisé par le fait qu'au premier stade, on mélange au moins une partie de l'isopropylidèneglycérol de formule (IV) et le catalyseur basique sous atmosphère inerte, on chauffe à une température comprise entre 50 et 150 ° C et on ajoute l'époxyde de formule (III).

**11.** Procédé selon l'une des revendications 6 à 10, caractérisé par le fait qu'au second stade, l'hydrolyse est effectuée en présence d'un catalyseur acide.

**12.** Procédé selon l'une des revendications 8 à 11, caractérisé par le fait que l'on effectue l'hydrolyse en présence d'un solvant.

**13.** Composés non-ioniques de formule II

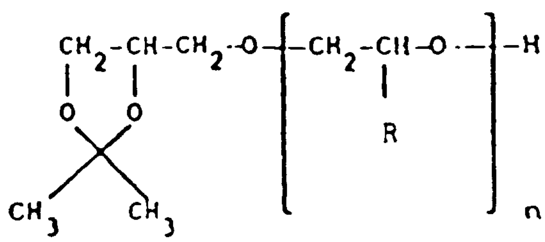

(II)

formule dans laquelle R et n ont la même signification que dans la revendication 1.

**14.** Composition cosmétique et/ou dermopharmaceutique, caractérisée par le fait qu'elle contient au moins un composé de formule I selon l'une des revendications 1 à 5.

**15.** Composition selon la revendication 14, caractérisée par le fait qu'elle se présente sous forme d'une composition huileuse, d'un gel, d'une cire, d'une émulsion eau-dans-l'huile ou huile-dans-l'eau.

**16.** Composition selon la revendication 14, caractérisée par le fait qu'elle renferme également d'autres agents tensioactifs ioniques ou non-ioniques, des polymères naturels ou synthétiques, ioniques ou non-ioniques, des huiles ou des cires, des protéines plus ou moins hydrolysées, des épaississants, des nacrants, des émollients, des hydratants, des colorants, des agents réducteurs ou oxydants, des conservateurs, des parfums, des filtres anti-UV, des solvants, des propulseurs ou des produits actifs pharmaceutiques ou parapharmaceutiques.

**17.** Composition selon la revendication 14, caractérisée par le fait qu'elle contient de 0,5 à 50 % et, de préférence de 0,5 à 25 % de composé de formule (I).

**18.** Composition selon la revendication 15, caractérisée par le fait qu'elle se présente sous forme d'une émulsion huile-dans-l'eau ou eau-dans-l'huile et qu'elle contient, en outre, des huiles ou cires et, éventuellement, d'autres agents tensioactifs émulsionnants différents de ceux de formule (I).

**19.** Composition selon la revendication 18, caractérisée par le fait que l'autre agent tensioactif émulsionnant, est constitué par les acides gras ou alcools gras polyoxyéthylénés, les alkyléthers de polyglycérol, les esters d'acide gras et de sorbitan polyoxyéthylénés ou non, les esters d'acides gras et de

sorbitol polyoxyéthylénés ou non, l'huile de ricin polyoxyéthylénée, les sels d'acides gras et d'amines ou de métaux polyvalents, les alkylsulfates polyoxyéthylénés ou non, et des alkylphosphates polyoxyéthylénés ou non.

**20.** Composition selon la revendication 14, caractérisée par le fait qu'elle contient au moins un actif cosmétique et/ou dermopharmaceutique.

**21.** Composition selon la revendication 14, caractérisée par le fait qu'elle contient, dispersées dans une phase aqueuse D, des vésicules délimitées par un ou plusieurs feuillets d'une phase lipidique contenant au moins un composé de formule I dans laquelle R représente un radical alkyle linéaire en $C_{14}$ - $C_{18}$ ou représente $-CH_2A$, A étant OR' et R' représentant un radical alkyle linéaire en $C_{10}$ - $C_{18}$ et n représente une valeur statistique moyenne $\overline{n}$ supérieure à 1 et au plus égale à 3 et, lorsque R est $-CH_2A$, est également égal à 2.

**22.** Composition selon la revendication 21, caractérisée par le fait que dans la phase lipidique, aux lipides amphiphiles non-ioniques de formule I, sont associées d'autres lipides ioniques et/ou lipides non-ioniques.

**23.** Composition selon l'une des revendications 21 ou 22, caractérisée par le fait que la phase lipidique des vésicules incorpore des additifs qui permettent de diminuer la perméabilité des vésicules et/ou des lipides chargés destinés à améliorer la stabilité des vésicules.

**24.** Composition selon la revendication 23, caractérisée par le fait que les additifs ou les lipides chargés sont choisis dans le groupe formé par les stérols et leurs dérivés, les alcools et diols à longue chaîne, les amines à longue chaîne et leurs dérivés ammonium quaternaire, les dihydroxyalkylamines, les amines grasses polyoxyéthylénées, les esters d'aminoalcools à longue chaîne et leurs sels et dérivés ammonium quaternaire, les esters phosphoriques d'alcool gras.

**25.** Composition selon l'une des revendications 21 à 24, caractérisée par le fait que les vésicules contiennent au moins un actif cosmétique et/ou dermopharmaceutique dans la phase lipidique et/ou dans la phase encapsulée.

**26.** Composition selon l'une des revendications 21 à 25, caractérisée par le fait que la phase aqueuse de dispersion des vésicules contient au moins un actif cosmétique et/ou dermopharmaceutique hydrosoluble et/ou au moins un actif amphiphile.

**27.** Composition selon la revendications 25, caractérisée par le fait que les parois des vésicules contiennent au moins un actif cosmétique et/ou dermopharmaceutique liposoluble.

**28.** Composition selon l'une des revendications 21 à 27, caractérisée par le fait que la phase aqueuse de dispersion contient une dispersion de gouttelettes d'un liquide non miscible à l'eau.

**29.** Composition selon la revendication 28, caractérisée par le fait que le liquide non miscible à l'eau contient au moins un actif cosmétique et/ou dermopharmaceutique liposoluble.

**30.** Composition selon la revendication 28, caractérisée par le fait que le liquide non miscible à l'eau est choisi dans le groupe formé par les huiles animales ou végétales formées par des esters d'acides gras et de polyols, les huiles essentielles, naturelles ou synthétiques, les hydrocarbures halogénés, les silicones, les esters d'acide minéral et d'un alcool, les éthers et les polyéthers.

**31.** Composition selon l'une des revendications 20 à 30, caractérisée par le fait que l'actif cosmétique et/ou dermopharmaceutique est choisi dans le groupe formé par les anti-oxydants ou les anti-radicaux libres, les agents hydratants ou humectants, les agents mélanorégulateurs accélérateur de bronzage, les agents mélanorégulateurs dépigmentant, les agents de coloration de la peau, les liporégulateurs, les agents anti-vieillissement et anti-rides, les agents anti-UV, les agents kératolytiques, les emollients, les agents anti-inflammatoires, les agents rafraîchissants, les agents cicatrisants, les agents protecteurs vasculaire, les agents anti-bactériens, les agents antifongiques les agents insectifuges, les agents antiperspirant, les agents déoderants, les agents anti-pelliculaires, les anti-chutes des cheveux, les

colorants capillaires, les agents décolorants pour cheveux, les réducteurs pour permanente, les agents conditionneurs pour la peau et les cheveux.

**32.** Composition selon l'une des revendications 14 à 31, caractérisée par le fait qu'elle contient au moins un additif de formulation n'ayant ni activité cosmétique, ni activité dermopharmaceutique.

**33.** Composition selon la revendication 32, caractérisée par le fait que l'additif de formulation est choisi parmi les gélifiants, les polymères, les conservateurs, les colorants, les opacifiants et les parfums.

**Revendications pour l'Etat contractant suivant : ES**

**1.** Procédé de préparation de composés amphiphiles non-ioniques dérivés du glycérol par un procédé en deux stades avec formation de produit intermédiaire, caractérisé par le fait que :

- dans un premier stade on fait réagir en présence d'un catalyseur basique de l'isopropylidène glycérol de formule (IV) avec un époxyde de formule (III) dans lequel R représente un radical pris dans le groupe formé par les radicaux alkyle ou alcényle, linéaires ou ramifiés en $C_4$-$C_{28}$ et leurs mélanges ou représente un groupement -$CH_2$A dans lequel A représente -OR', -SR' ou

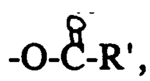

R' représentant un radical hydrocarboné saturé ou insaturé pour obtetir un (des) produit(s) intermédiaire(s) de formule (II) selon le schéma de réaction suivant :

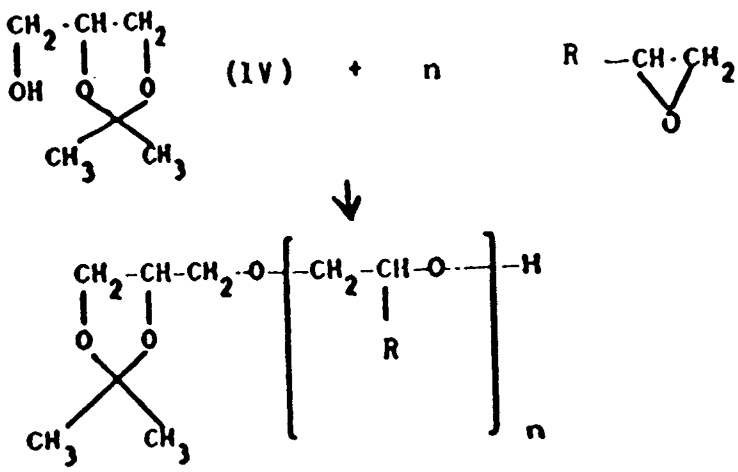

(IV) + n (III)

(II)

dans lequel R a la même signification que ci-dessus et n représente une valeur statistique moyenne $\bar{n}$ supérieure à 1 et au plus égale à 6 et lorsque R est $CH_2$A représente également une valeur égale à 2,

- dans un second stade, on hydrolyse le (les) produit(s) intermédiaire(s) de formule (II) obtenu(s) et l'on sépare un (des) composé(s) de formule (I) :

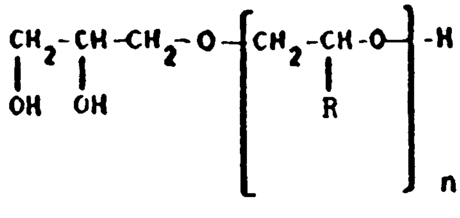

(I)

formule (I) dans laquelle R et n ont la même valeur que ci-dessus.

2. Procédé selon la revendication 1, caractérisé par le fait que le catalyseur basique utilisé au premier stade est choisi dans le groupe formé par les métaux alcalins, les hydrures de métaux alcalins, les hydroxydes alcalins, les alcoolates alcalins et les fluorures de métaux alcalins.

3. Procédé selon la revendication 2, caractérisé par le fait que le catalyseur basique est le tert-butylate de potassium.

4. Procédé selon l'une des revendications 1 à 3, caractérisé par le fait que la quantité de catalyseur basique utilisée est comprise entre 4 et 40 % en moles par rapport à l'isopropylidèneglycérol de formule (IV).

5. Procédé selon l'une des revendications 1 à 4, caractérisé par le fait qu'au premier stade, on mélange au moins une partie de l'isopropylidèneglycérol de formule (IV) et le catalyseur basique sous atmosphère inerte, on chauffe à une température comprise entre 50 et 150 °C et on ajoute l'époxyde de formule (III).

6. Procédé selon l'une des revendications 1 à 5, caractérisé par le fait qu'au second stade, l'hydrolyse est effectuée en présence d'un catalyseur acide.

7. Procédé selon l'une des revendications 3 à 6, caractérisé par le fait que l'on effectue l'hydrolyse en présence d'un solvant.

8. Composition cosmétique caractérisée par le fait qu'elle contient au moins un composé de formule (I)

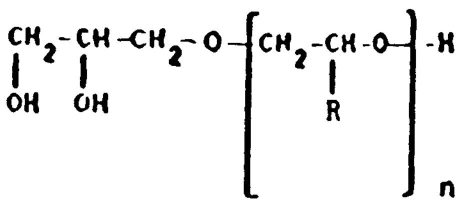 (I)

dans laquelle :

- R représente un radical pris dans le groupe formé par les radicaux alkyle ou alcényle, linéaires ou ramifiés, en $C_4$-$C_{28}$ et leurs mélanges ou représente un groupement -$CH_2$A dans lequel A représente -OR', -SR' ou

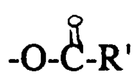,

  R' représentant un radical hydrocarboné saturé ou insaturé et
- n représente une valeur statistique moyenne $\bar{n}$ supérieure à 1 et au plus égale à 6 et, lorsque R est -$CH_2$A, représente également une valeur égale à 2.

9. Composition selon la revendication 8, caractérisée par le fait qu'elle contient au moins un composé de formule (I) dans lequel le radical hydrocarboné R' est un radical alkyle linéaire en $C_8$-$C_{22}$, un radical alkyle ramifié en $C_8$-$C_{36}$, un radical alcényle en $C_{18}$ ou un radical alkylaryle à chaîne alkyle linéaire ou ramifié en $C_8$-$C_{16}$.

10. Composition selon la revendication 9 contenant le composé de formule (I) dans lequel R' est un radical alkylaryle, caractérisée par le fait que le radical aryle est un radical phényle.

11. Composition selon la revendication 9 contenant un composé de formule (I) dans lequel R est un radical alcényle, caractérisée par le fait que le radical alcényle est un radical octadécène-9 yle ou octadécanediène-9,12 yle.

12. Composition selon la revendication 8, caractérisée par le fait qu'elle contient un composé de formule (I) dans lequel R représente un radical alkyle linéaire en $C_{14}$-$C_{18}$ ou un groupement $-CH_2A$ dans lequel A est OR', R' représentant un radical alkyle linéaire en $C_{10}$-$C_{18}$ et n représente une valeur statistique moyenne $\bar{n}$ supérieure à 1 et au plus égale à 3 et, lorsque R représente $-CH_2A$ est également égal à 2.

13. Composition selon la revendication 8, caractérisée par le fait qu'elle se présente sous forme d'une composition huileuse, d'un gel, d'une cire, d'une émulsion eau-dans-l'huile ou huile-dans-l'eau.

14. Composition selon la revendication 8, caractérisée par le fait qu'elle renferme également d'autres agents tensioactifs ioniques ou non-ioniques, des polymères naturels ou synthétiques, ioniques ou non-ioniques, des huiles ou des cires, des protéines plus ou moins hydrolysées, des épaissisants, des nacrants, des émollients, des hydratants, des colorants, des agents réducteurs ou oxydants, des conservateurs, des parfums, des filtres anti-UV, des solvants, des propulseurs ou des produits actifs pharmaceutiques ou parapharmaceutiques.

15. Composition selon la revendication 8, caractérisée par le fait qu'elle contient de 0,5 à 50 % et, de préférence de 0,5 à 25 % de composé de formule (I).

16. Composition selon la revendication 13, caractérisée par le fait qu'elle se présente sous forme d'une émulsion huile-dans-l'eau ou eau-dans-l'huile et qu'elle contient, en outre, des huiles ou cires et, éventuellement, d'autres agents tensioactifs émulsionnants différents de ceux de formule (I).

17. Composition selon la revendication 16, caractérisée par le fait que l'autre agent tensioactif émulsionnant, est constitué par les acides gras ou alcools gras polyoxyéthylénés, les alkyléthers de polyglycérol, les esters d'acide gras et de sorbitan polyoxyéthylénés ou non, les esters d'acides gras et de sorbitol polyoxyéthylénés ou non, l'huile de ricin polyoxyéthylénée, les sels d'acides gras et d'amines ou de métaux polyvalents, les alkylsulfates polyoxyéthylénés ou non, et des alkylphosphates polyoxyéthylénés ou non.

18. Composition selon la revendication 8, caractérisée par le fait qu'elle contient au moins un actif cosmétique et/ou dermopharmaceutique.

19. Composition selon la revendication 8, caractérisée par le fait qu'elle contient, dispersées dans une phase aqueuse D, des vésicules délimitées par un ou plusieurs feuillets d'une phase lipidique contenant au moins un composé de formule I dans laquelle R représente un radical alkyle linéaire en $C_{14}$-$C_{18}$ ou représente $-CH_2A$, A étant OR' et R' représentant un radical alkyle linéaire en $C_{10}$-$C_{18}$ et n représente une valeur statistique moyenne $\bar{n}$ supérieure à 1 et au plus égale à 3 et, lorsque R est $-CH_2A$, est également égal à 2.

20. Composition selon la revendication 19, caractérisée par le fait que dans la phase lipidique, aux lipides amphiphiles non-ioniques de formule I, sont associées d'autres lipides ioniques et/ou lipides non-ioniques.

21. Composition selon l'une des revendications 19 ou 20, caractérisée par le fait que la phase lipidique des vésicules incorpore des additifs qui permettent de diminuer la perméabilité dès vésicules et/ou des lipides chargés destinés à améliorer la stabilité des vésicules.

22. Composition selon la revendication 21, caractérisée par le fait que les additifs ou les lipides chargés sont choisis dans le groupe formé par les stérols et leurs dérivés, les alcools et diols à longue chaîne, les amines à longue chaîne et leurs dérivés ammonium quaternaire, les dihydroxyalkylamines, les amines grasses polyoxyéthylénées, les esters d'aminoalcools à longue chaîne et leurs sels et dérivés ammonium quaternaire, les esters phosphoriques d'alcool gras.

23. Composition selon l'une des revendications 19 à 22, caractérisée par le fait que les vésicules contiennent au moins un actif cosmétique et/ou dermopharmaceutique dans la phase lipidique et/ou dans la phase encapsulée.

**24.** Composition selon l'une des revendications 19 à 23, caractérisée par le fait que la phase aqueuse de dispersion des vésicules contient au moins un actif cosmétique et/ou dermopharmaceutique hydrosoluble et/ou au moins un actif amphiphile.

**25.** Composition selon la revendication 23, caractérisée par le fait que les parois des vésicules contiennent au moins un actif cosmétique et/ou dermopharmaceutique liposoluble.

**26.** Composition selon l'une des revendications 19 à 25, caractérisée par le fait que la phase aqueuse de dispersion contient une dispersion de gouttelettes d'un liquide non miscible à l'eau.

**27.** Composition selon la revendication 26, caractérisée par le fait que le liquide non miscible à l'eau contient au moins un actif cosmétique et/ou dermopharmaceutique liposoluble.

**28.** Composition selon la revendication 26, caractérisée par le fait que le liquide non miscible à l'eau est choisi dans le groupe formé par les huiles animales ou végétales formées par des esters d'acides gras et de polyols, les huiles essentielles, naturelles ou synthétiques, les hydrocarbures halogénés, les silicones, les esters d'acide minéral et d'un alcool, les éthers et les polyéthers.

**29.** Composition selon l'une des revendications 18 à 28, caractérisée par le fait que l'actif cosmétique et/ou dermopharmaceutique est choisi dans le groupe formé par les anti-oxydants ou les anti-radicaux libres, les agents hydratants ou humectants, les agents mélanorégulateurs accélérateur de bronzage, les agents mélanorégulateurs dépigmetant, les agents de coloration de la peau, les liporégulateurs, les agents anti-vieillissement et anti-rides les agents anti-UV, les agents kératolytiques, les emollients, les agents anti-inflammatoires, les agents rafraîchissants, les agents cicatrisants, les agents protecteurs vasculaire, les agents anti-bactériens, les agents antifongiques, les agents insectifuges, les agents antiperspirant, les agents déodorants, les agents anti-pelliculaires, les anti-chutes des cheveux, les colorants capillaires, les agents décolorants pour cheveux, les réducteurs pour permanente, les agents conditionneurs pour la peau et les cheveux.

**30.** Composition selon l'une des revendications 12 à 28, caractérisée par le fait qu'elle contient au moins un additif de formulation n'ayant ni activité cosmétique, ni activité dermopharmaceutique.

**31.** Composition selon la revendication 30, caractérisée par le fait que l'additif de formulation est choisi parmi les gélifiants, les polymères, les conservateurs, les colorants, les opacifiants et les parfums.

**Revendications pour l'Etat contractant suivant : GR**

**1.** Composés amphiphiles non-ioniques dérivés du glycérol, caractérisés par le fait qu'ils ont la formule (I):

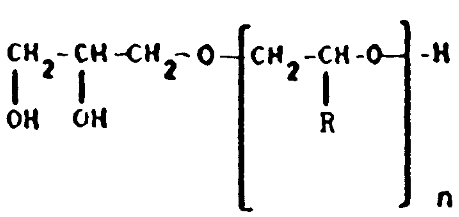 (I)

formule dans laquelle :

- R représente un radical pris dans le groupe formé par les radicaux alkyle ou alcényle, linéaires ou ramifiés, en $C_4$ - $C_{28}$ et leurs mélanges ou représente un groupement $-CH_2A$ dans lequel A représente -OR', -SR' ou

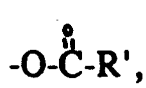

R' représentant un radical hydrocarboné saturé ou insaturé et

- n représente une valeur statistique moyenne $\bar{n}$ supérieure à 1 et au plus égale à 6 et, lorsque R est $-CH_2A$, représente également une valeur égale à 2.

**2.** Composé selon la revendication 1, caractérisé par le fait que le radical hydrocarboné R' est un radical alkyle linéaire en $C_8$ - $C_{22}$, un radical alkyle ramifié en $C_8$ - $C_{36}$, un radical alcényle en $C_{18}$ ou un radical alkylaryle à chaîne alkyle linéaire ou ramifié en $C_8$ - $C_{16}$.

**3.** Composé selon la revendication 2, caractérisé par le fait que dans le radical alkylaryle le groupe aryle est un radical phényle.

**4.** Composé selon la revendication 2, caractérisé par le fait que le radical alcényle est un radical octadécène-9 yle ou octadécanediène 9,12 yle.

**5.** Composé selon la revendication 1, caractérisé par le fait que le radical R représente un radical alkyle linéaire en $C_{14}$ - $C_{18}$ ou un groupement $-CH_2A$ dans lequel A est OR', R' représentant un radical alkyle linéaire en $C_{10}$ - $C_{18}$ et n représente une valeur statistique moyenne $\bar{n}$ supérieure à 1 et au plus égale à 3 et, lorsque R représente $-CH_2A$ est également égal à 2.

**6.** Procédé de préparation de composés de formule I selon l'une des revendications 1 à 5 par un procédé en deux stades avec formation de produit intermédiaire, caractérisé par le fait que :

- dans un premier stade, on fait réagir, en présence d'un catalyseur basique, de l'isopropylidèneglycérol de formule (IV) avec un époxyde de formule (III) dans lequel
  R a la même signification que dans la formule (I), pour obtenir un (des) produit(s) intermédiaire(s) de formule (II) selon le schéma de réaction suivant :

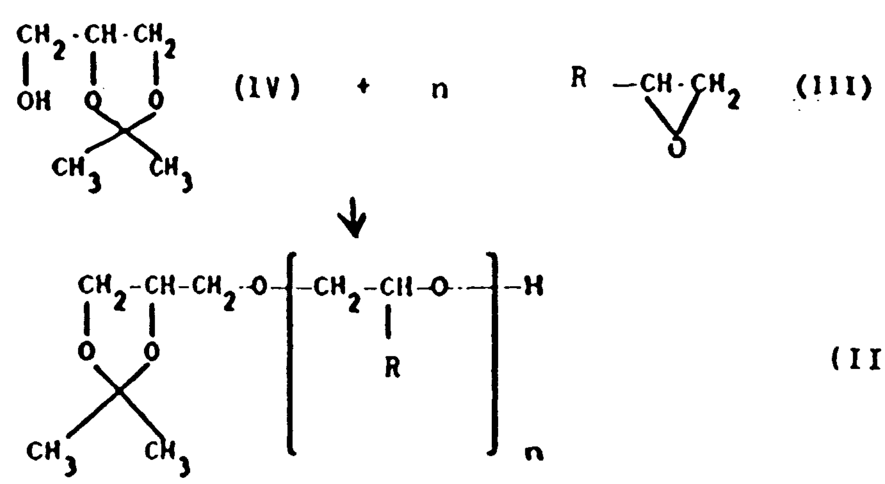

(IV) + n (III)

(II)

  R et n ayant la même signification que dans la formule (I)
  et
- dans un second stade, on hydrolyse le (les) produit(s) intermédiaire(s) de formule (II) obtenu(s) et l'on sépare le (les) composé(s) de formule (I) du mélange réactionnel.

**7.** Procédé selon la revendication 6, caractérisé par le fait que le catalyseur basique utilisé au premier stade est choisi dans le groupe formé par les métaux alcalins, les hydrures de métaux alcalins, les hydroxydes alcalins, les alcoolates alcalins et les fluorures de métaux alcalins.

**8.** Procédé selon la revendication 7, caractérisé par le fait que le catalyseur basique est le tert-butylate de potassium.

**9.** Procédé selon l'une des revendications 6 à 8, caractérisé par le fait que la quantité de catalyseur basique utilisée est comprise entre 4 et 40 % en moles par rapport à l'isopropylidèneglycérol de formule (IV).

**10.** Procédé selon l'une des revendications 6 à 9, caractérisé par le fait qu'au premier stade, on mélange au moins une partie de l'isopropylidèneglycérol de formule (IV) et le catalyseur basique sous atmosphère inerte, on chauffe à une température comprise entre 50 et 150 ° C et on ajoute l'époxyde de formule (III).

**11.** Procédé selon l'une des revendications 6 à 10, caractérisé par le fait qu'au second stade, l'hydrolyse est effectuée en présence d'un catalyseur acide.

**12.** Procédé selon l'une des revendications 8 à 11, caractérisé par le fait que l'on effectue l'hydrolyse en présence d'un solvant.

**13.** Composés non-ioniques de formule II

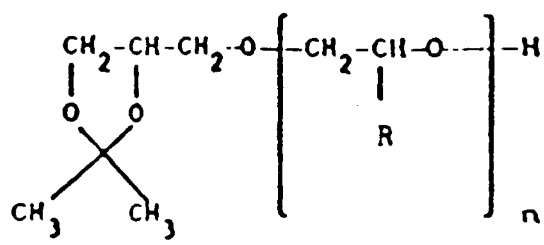

(II)

formule dans laquelle R et n ont la même signification que dans la revendication 1.

**14.** Composition cosmétique, caractérisée par le fait qu'elle contient au moins un composé de formule I selon l'une des revendications 1 à 5.

**15.** Composition selon la revendication 14, caractérisée par le fait qu'elle se présente sous forme d'une composition huileuse, d'un gel, d'une cire, d'une émulsion eau-dans-l'huile ou huile-dans-l'eau.

**16.** Composition selon la revendication 14, caractérisée par le fait qu'elle renferme également d'autres agents tensioactifs ioniques ou non-ioniques, des polymères naturels ou synthétiques, ioniques ou non-ioniques, des huiles ou des cires, des protéines plus ou moins hydrolysées, des épaissisants, des nacrants, des émollients, des hydratants, des colorants, des agents réducteurs ou oxydants, des conservateurs, des parfums, des filtres anti-UV, des solvants, des propulseurs ou des produits actifs pharmaceutiques ou parapharmaceutiques.

**17.** Composition selon la revendication 14, caractérisée par le fait qu'elle contient de 0,5 à 50 % et, de préférence de 0,5 à 25 % de composé de formule (I).

**18.** Composition selon la revendication 15, caractérisée par le fait qu'elle se présente sous forme d'une émulsion huile-dans-l'eau ou eau-dans-l'huile et qu'elle contient, en outre, des huiles ou cires et, éventuellement, d'autres agents tensioactifs émulsionnants différents de ceux de formule (I).

**19.** Composition selon la revendication 18, caractérisée par le fait que l'autre agent tensioactif émulsionnant, est constitué par les acides gras ou alcools gras polyoxyéthylénés, les alkyléthers de polyglycérol, les esters d'acide gras et de sorbitan polyoxyéthylénés ou non, les esters d'acides gras et de sorbitol polyoxyéthylénés ou non, l'huile de ricin polyoxyéthylénée, les sels d'acides gras et d'amines ou de métaux polyvalents, les alkylsulfates polyoxyéthylénés ou non, et des alkylphosphates polyoxyéthylénés ou non.

**20.** Composition selon la revendication 14, caractérisée par le fait qu'elle contient au moins un actif cosmétique et/ou dermopharmaceutique.

**21.** Composition selon la revendication 14, caractérisée par le fait qu'elle contient, dispersées dans une phase aqueuse D, des vésicules délimitées par un ou plusieurs feuillets d'une phase lipidique contenant au moins un composé de formule I dans laquelle R représente un radical alkyle linéaire en $C_{14}$ - $C_{18}$ ou représente $-CH_2A$, A étant OR' et R' représentant un radical alkyle linéaire en $C_{10}$ - $C_{18}$ et

n représente une valeur statistique moyenne $\bar{n}$ supérieure à 1 et au plus égale à 3 et, lorsque R est $-CH_2A$, est également égal à 2.

**22.** Composition selon la revendication 21, caractérisée par le fait que dans la phase lipidique, aux lipides amphiphiles non-ioniques de formule I, sont associées d'autres lipides ioniques et/ou lipides non-ioniques.

**23.** Composition selon l'une des revendications 21 ou 22, caractérisée par le fait que la phase lipidique des vésicules incorpore des additifs qui permettent de diminuer la perméabilité des vésicules et/ou des lipides chargés destinés à améliorer la stabilité des vésicules.

**24.** Composition selon la revendication 23, caractérisée par le fait que les additifs ou les lipides chargés sont choisis dans le groupe formé par les stérols et leurs dérivés, les alcools et diols à longue chaîne, les amines à longue chaîne et leurs dérivés ammonium quaternaire, les dihydroxyalkylamines, les amines grasses polyoxyéthylénées, les esters d'aminoalcools à longue chaîne et leurs sels et dérivés ammonium quaternaire, les esters phosphoriques d'alcool gras.

**25.** Composition selon l'une des revendications 21 à 24, caractérisée par le fait que les vésicules contiennent au moins un actif cosmétique et/ou dermopharmaceutique dans la phase lipidique et/ou dans la phase encapsulée.

**26.** Composition selon l'une des revendications 21 à 25, caractérisée par le fait que la phase aqueuse de dispersion des vésicules contient au moins un actif cosmétique et/ou dermopharmaceutique hydrosoluble et/ou au moins un actif amphiphile.

**27.** Composition selon la revendications 25, caractérisée par le fait que les parois des vésicules contiennent au moins un actif cosmétique et/ou dermopharmaceutique liposoluble.

**28.** Composition selon l'une des revendications 21 à 27, caractérisée par le fait que la phase aqueuse de dispersion contient une dispersion de gouttelettes d'un liquide non miscible à l'eau.

**29.** Composition selon la revendication 28, caractérisée par le fait que le liquide non miscible à l'eau contient au moins un actif cosmétique et/ou dermopharmaceutique liposoluble.

**30.** Composition selon la revendication 28, caractérisée par le fait que le liquide non miscible à l'eau est choisi dans le groupe formé par les huiles animales ou végétales formées par des esters d'acides gras et de polyols, les huiles essentielles, naturelles ou synthétiques, les hydrocarbures halogénés, les silicones, les esters d'acide minéral et d'un alcool, les éthers et les polyéthers.

**31.** Composition selon l'une des revendications 20 à 30, caractérisée par le fait que l'actif cosmétique et/ou dermopharmaceutique est choisi dans le groupe formé par les anti-oxydants ou les anti-radicaux libres, les agents hydratants ou humectants, les agents mélanorégulateurs accélérateur de bronzage, les agents mélanorégulateurs dépigmentant, les agents de coloration de la peau, les liporégulateurs, les agents anti-vieillissement et anti-rides, les agents anti-UV, les agents kératolytiques, les emollients, les agents anti-inflammatoires, les agents rafraîchissants, les agents cicatrisants, les agents protecteurs vasculaire, les agents anti-bactériens, les agents antifongiques, les agents insectifuges, les agents antiperspirant, les agents déodorants, les agents anti-pelliculaires, les anti-chutes des cheveux, les colorants capillaires, les agents décolorants pour cheveux, les réducteurs pour permanente, les agents conditionneurs pour la peau et les cheveux.

**32.** Composition selon l'une des revendications 14 à 31, caractérisée par le fait qu'elle contient au moins un additif de formulation n'ayant ni activité cosmétique, ni activité dermopharmaceutique.

**33.** Composition selon la revendication 32, caractérisée par le fait que l'additif de formulation est choisi parmi les gélifiants, les polymères, les conservateurs, les colorants, les opacifiants et les parfums.

## Claims

**Claims for the following Contracting States : AT, BE, CH, DE, DK, FR, GB, IT, LI, LU, NL, SE**

**1.** Nonionic amphiphilic compounds derived from glycerol, characterized in that they are of the formula (I):

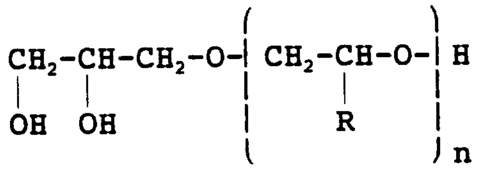

(I)

in which formula:

R represents a radical taken from the group composed of linear or branched $C_4$-$C_{28}$ alkyl or alkenyl radicals and mixtures thereof, or represents a group $-CH_2A$ in which A represents -OR',-SR' or

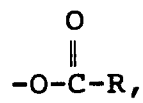

R' representing a saturated or unsaturated hydrocarbon radical, and

- n represents an average statistical value $\bar{n}$ greater than 1 and equal to not more than 6 and, when R is $-CH_2A$, also represents a value equal to 2.

**2.** Compound according to Claim 1, characterized in that the hydrocarbon radical R' is a linear $C_8$-$C_{22}$ alkyl radical, a branched $C_8$-$C_{36}$ alkyl radical, a $C_{18}$ alkenyl radical or an alkylaryl radical having a linear or branched $C_8$-$C_{16}$ alkyl chain.

**3.** Compound according to Claim 2, characterized in that, in the alkylaryl radical, the aryl group is a phenyl radical.

**4.** Compound according to Claim 2, characterized in that the alkenyl radical is a 9-octadecenyl or 9,12-octadecanedienyl radical.

**5.** Compound according to Claim 1, characterized in that the radical R represents a linear $C_{14}$-$C_{18}$ alkyl radical or a group $-CH_2A$ in which A is OR', R' representing a linear $C_{10}$-$C_{18}$ alkyl radical, and n represents an average statistical value $\bar{n}$ greater than 1 and equal to not more than 3 and, when R represents $-CH_2A$, is also equal to 2.

**6.** Process for preparing compounds of formula I according to one of Claims 1 to 5, by a two-stage process with formation of intermediate product, characterized in that :

- in a first stage, isopropylideneglycerol of formula (IV) is reacted in the presence of a basic catalyst with an epoxide of formula (III), in which R has the same meaning as in the formula (I), to obtain one or more intermediate product(s) of formula (II) according to the following reaction scheme:

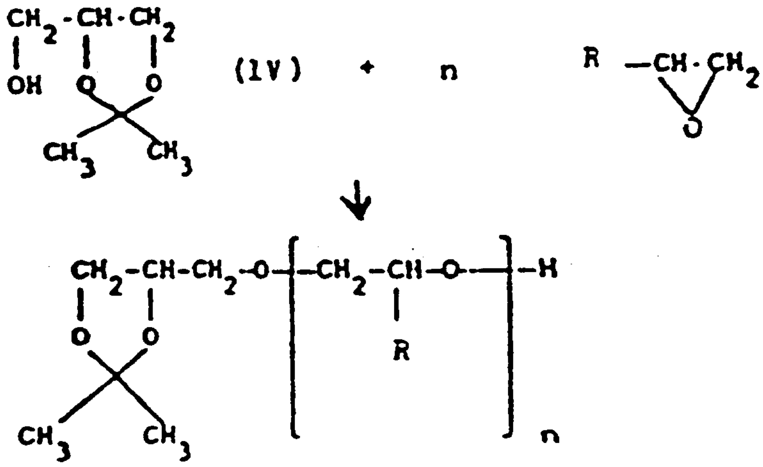

(IV) + n (III)

(II)

R and n having the same meaning as in the formula (I), and

- in a second stage, the intermediate product(s) of formula (II) obtained is/are hydrolyzed and the compound(s) of formula (I) is/are separated from the reaction mixture.

**7.** Process according to Claim 6, characterized in that the basic catalyst used in the first stage is chosen from the group composed of alkali metals, alkali metal hydrides, alkali metal hydroxides, alkali metal alcoholates and alkali metal fluorides.

**8.** Process according to Claim 7, characterized in that the basic catalyst is potassium tertbutylate.

**9.** Process according to one of Claims 6 to 8, characterized in that the amount of basic catalyst used is between 4 and 40 mol % relative to the isopropylideneglycerol of formula (IV).

**10.** Process according to one of Claims 6 to 9, characterized in that, in the first stage, at least a part of the isopropylideneglycerol of formula (IV) and the basic catalyst are mixed under an inert atmosphere, the mixture is heated to a temperature of between 50 and 150 °C and the epoxide of formula (III) is added.

**11.** Process according to one of Claims 6 to 10, characterized in that, in the second stage, the hydrolysis is performed in the presence of an acid catalyst.

**12.** Process according to one of Claims 8 to 11, characterized in that the hydrolysis is performed in the presence of a solvent.

**13.** Nonionic compounds of formula II

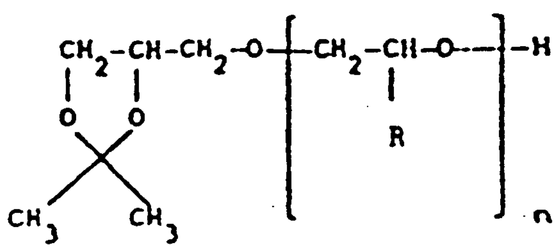

(II)

in which R and n have the same meaning as in Claim 1.

**14.** Cosmetic and/or dermopharmaceutical composition, characterized in that it contains at least one compound of formula I according to one of Claims 1 to 5.

**15.** Composition according to Claim 14, characterized in that it takes the form of an oily composition, a gel, a wax or a water-in-oil or oil-in-water emulsion.

**16.** Composition according to Claim 14, characterized in that it also contains other ionic or nonionic surfactant agents, natural or synthetic, ionic or nonionic polymers, oils or waxes, more or less hydrolyzed proteins, thickeners, pearlescent agents, emollients, hydrating agents, colorants, reducing or oxidizing agents, preservatives, perfumes, anti-UV screening agents, solvents, propellants or pharmaceutical or parapharmaceutical active products.

**17.** Composition according to Claim 14, characterized in that it contains 0.5 to 50 % and preferably from 0.5 to 25 %, of compound of formula (I).

**18.** Composition according to Claim 15, characterized in that it takes the form of an oil-in-water or water-in-oil emulsion and in that it contains, in addition, oils or waxes and, where appropriate, other emulsifying surfactant agents different from those of formula (I).

**19.** Composition according to Claim 18, characterized in that the other emulsifying surfactant agent consists of polyoxyethylenated fatty acids or fatty alcohols, polyglycerol alkyl ethers, esters of fatty acid and sorbitan, polyoxyethylenated or otherwise, esters of fatty acids and sorbitol, polyoxyethylenated or otherwise, polyoxyethylenated castor oil, salts of fatty acids and amines or polyvalent metals, alkyl sulfates, polyoxyethylenated or otherwise, and alkyl phosphates polyoxyethylenated or otherwise.

**20.** Composition according to Claim 14, characterized in that it contains at least one cosmetic and/or dermopharmaceutical active compound.

**21.** Composition according to Claim 14, characterized in that it contains, dispersed in an aqueous phase D, vesicles bounded by one or more lamellae of a lipid phase containing at least one compound of formula I in which R represents a linear $C_{14}$-$C_{18}$ alkyl radical or represents -$CH_2A$, A being OR' and R' representing a linear $C_{10}$-$C_{18}$ alkyl radical, and n represents an average statistical value $\overline{n}$ greater than 1 and equal to not more than 3 and, when R is -$CH_2A$, is also equal to 2.

**22.** Composition according to Claim 21, characterized in that, in the lipid phase, other ionic lipids and/or nonionic lipids are combined with the nonionic amphiphilic lipids of formula I.

**23.** Composition according to one of Claims 21 and 22, characterized in that the lipid phase of the vesicles incorporates additives which enable the permeability of the vesicles to be decreased, and/or charged lipids intended for improving the stability of the vesicles.

**24.** Composition according to Claim 23, characterized in that the additives or charged lipids are chosen from the group composed of sterols and their derivatives, long-chain alcohols and diols, long-chain amines and their quaternary ammonium derivatives, dihydroxyalkylamines, polyoxyethylenated fatty amines, eaters of long-chain amino alcohols and their salts and quaternary ammonium derivatives, and phosphoric esters of fatty alcohols.

**25.** Composition according to one of Claims 21 to 24, characterized in that the vesicles contain at least one cosmetic and/or dermopharmaceutical active compound in the lipid phase and/or in the encapsulated phase.

**26.** Composition according to one of Claims 21 to 25, characterized in that the aqueous phase of dispersion of the vesicles contains at least one water-soluble cosmetic and/or dermopharmaceutical cosmetic active compound and/or at least one amphiphilic active compound.

**27.** Compound according to Claim 25, characterized in that the walls of the vesicles contain at least one fat-soluble cosmetic and/or dermopharmaceutical active compound.

**28.** Composition according to one of Claims 21 to 27, characterized in that the aqueous dispersion phase contains a dispersion of droplets of a water-immiscible liquid.

**29.** Composition according to Claim 28, characterized in that the water-immiscible liquid contains at least one fat-soluble cosmetic and/or dermopharmaceutical active compound.

**30.** Composition according to Claim 28, characterized in that the water-immiscible liquid is chosen from the group composed of animal or vegetable oils composed of esters of fatty acids and polyols, natural or synthetic essential oils, halogenated hydrocarbons, silicones, eaters of an inorganic acid and an alcohol, ethers and polyethers.

**31.** Composition according to one of Claims 20 to 30, characterized in that the cosmetic and/or dermopharmaceutical active compound is chosen from the group composed of antioxidants or free-radical inhibitors, hydrating or humectant agents, tanning accelerator melanoregulatory agents, depigmenting melanoregulatory agents, skin coloration agents, liporegulators, anti-aging and anti-wrinkle agents, anti-UV agents, keratolytic agents, emollients, anti-inflammatory agents, refreshing agents, cicatrizing agents, vasoprotective agents, antibacterial agents, antifungal agents, insect-repellant agents, antiperspirant agents, deodorant agents, anti-dandruff agents, agents for combating hair loss, hair dyes, hair bleaching agents, reducing agents for permanent waving and skin and hair conditioners.

**32.** Composition according to one of Claims 14 to 31, characterized in that it contains at least one formulation additive having neither cosmetic activity nor dermopharmaceutical activity.

**33.** Composition according to Claim 32, characterized in that the formulation additive is chosen from gelling agents, polymers, preservatives, colorants, opacifiers and perfumes.

**Claims for the following Contracting State : ES**

**1.** Process for preparing nonionic amphiphilic compounds derived from glycerol, by a two-stage process with formation of intermediate product, characterized in that :

- in a first stage, isopropylideneglycerol of formula (IV) is reacted in the presence of a basic catalyst with an epoxide of formula (III), in which R represents a radical taken from the group composed of linear or branched $C_4$-$C_{28}$ alkyl or alkenyl radicals and mixtures thereof, or represents a group $-CH_2A$ in which A represents -OR',-SR' or

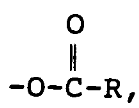

R' representing a saturated or unsaturated hydrocarbon radical, to obtain one or more intermediate product(s) of formula (II) according to the following reaction scheme:

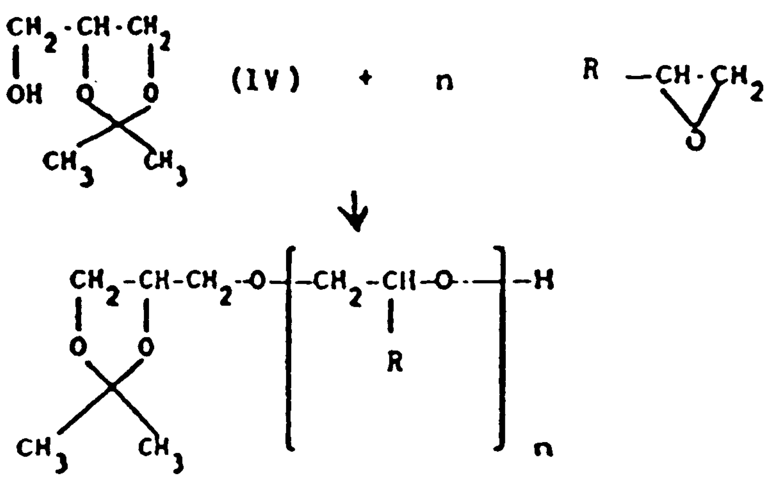
(IV) + n (III)

(II)

in which R has the same meaning as above and n represents an average statistical value $\bar{n}$

greater than 1 and equal to not more than 6 and when R is $CH_2A$, also represents a value equal to 2,

- in a second stage, the intermediate product(s) of formula (II) obtained is/are hydrolyzed and (a) compound(s) of formula (I):

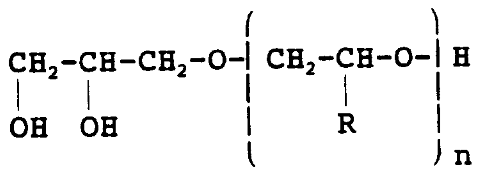

(I)

is/are separated, in which formula (I) R and n have the same meaning as above.

**2.** Process according to Claim 1, characterized in that the basic catalyst used in the first stage is chosen from the group composed of alkali metals, alkali metal hydrides, alkali metal hydroxides, alkali metal alcoholates and alkali metal fluorides.

**3.** Process according to Claim 2, characterized in that the basic catalyst is potassium tertbutylate.

**4.** Process according to one of Claims 1 to 3, characterized in that the amount of basic catalyst used is between 4 and 40 mol % relative to the isopropylideneglycerol of formula (IV).

**5.** Process according to one of Claims 1 to 4, characterized in that, in the first stage, at least a part of the isopropylideneglycerol of formula (IV) and the basic catalyst are mixed under an inert atmosphere, the mixture is heated to a temperature of between 50 and 150 °C and the epoxide of formula (III) is added.

**6.** Process according to one of Claims 1 to 5, characterized in that, in the second stage, the hydrolysis is performed in the presence of an acid catalyst.

**7.** Process according to one of Claims 3 to 6, characterized in that the hydrolysis is performed in the presence of a solvent.

**8.** Cosmetic composition, characterized in that it contains at least one compound of formula (I)

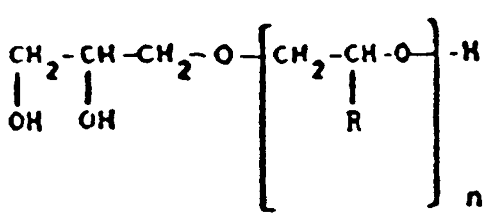

(I)

in which:

- R represents a radical taken from the group composed of linear or branched $C_4$-$C_{28}$ alkyl or alkenyl radicals and mixtures thereof, or represents a group $-CH_2A$ in which A represents -OR', -SR' or

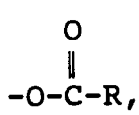

R' representing a saturated or unsaturated hydrocarbon radical, and

- n represents an average statistical value $\bar{n}$ greater than 1 and equal to not more than 6 and, when R is $-CH_2A$, also represents a value equal to 2.

**9.** Composition according to Claim 8, characterized in that it contains at least one compound of formula (I) in which the hydrocarbon radical R' is a linear $C_8$-$C_{22}$ alkyl radical, a branched $C_8$-$C_{36}$ alkyl radical, a $C_{18}$ alkenyl radical or an alkylaryl radical having a linear or branched $C_8$-$C_{16}$ alkyl chain.

**10.** Composition according to Claim 9 containing the compound of formula (I) in which R' is an alkylaryl radical, characterized in that the aryl radical is a phenyl radical.

**11.** Composition according to Claim 9 containing a compound of formula (I) in which R is an alkenyl radical, characterized in that the alkenyl radical is a 9-octadecenyl or 9,12-octadecanedienyl radical.

**12.** Composition according to Claim 8, characterized in that it contains a compound of formula (I) in which R represents a linear $C_{14}$-$C_{18}$ alkyl radical or a group $-CH_2A$ in which A is OR', R' representing a linear $C_{10}$-$C_{18}$ alkyl radical, and n represents an average statistical value $\bar{n}$ greater than 1 and equal to not more than 3 and, when R represents $-CH_2A$, is also equal to 2.

**13.** Composition according to Claim 8, characterized in that it takes the form of an oily composition, a gel, a wax or a water-in-oil or oil-in-water emulsion.

**14.** Composition according to Claim 8, characterized in that it also contains other ionic or nonionic surfactant agents, natural or synthetic, ionic or nonionic polymers, oils or waxes, more or less hydrolyzed proteins, thickeners, pearlescent agents, emollients, hydrating agents, colorants, reducing or oxidizing agents, preservatives, perfumes, anti-UV screening agents, solvents, propellants or pharmaceutical or parapharmaceutical active products.

**15.** Composition according to Claim 8, characterized in that it contains 0.5 to 50 % and preferably from 0.5 to 25 %, of compound of formula (I).

**16.** Composition according to Claim 13, characterized in that it takes the form of an oil-in-water or water-in-oil emulsion and in that it contains, in addition, oils or waxes and, where appropriate, other emulsifying surfactant agents different from those of formula (I).

**17.** Composition according to Claim 16, characterized in that the other emulsifying surfactant agent consists of polyoxyethylenated fatty acids or fatty alcohols, polyglycerol alkyl ethers, esters of fatty acid and sorbitan, polyoxyethylenated or otherwise, esters of fatty acids and sorbitol, polyoxyethylenated or otherwise, polyoxyethylenated castor oil, salts of fatty acids and amines or polyvalent metals, alkyl sulfates, polyoxyethylenated or otherwise, and alkyl phosphates polyoxyethylenated or otherwise.

**18.** Composition according to Claim 8, characterized in that it contains at least one cosmetic and/or dermopharmaceutical active compound.

**19.** Composition according to Claim 8, characterized in that it contains, dispersed in an aqueous phase D, vesicles bounded by one or more lamellae of a lipid phase containing at least one compound of formula I in which R represents a linear $C_{14}$-$C_{18}$ alkyl radical or represents $-CH_2A$, A being OR' and R' representing a linear $C_{10}$-$C_{18}$ alkyl radical, and n represents an average statistical value $\bar{n}$ greater than 1 and equal to not more than 3 and, when R is $-CH_2A$, is also equal to 2.

**20.** Composition according to Claim 19, characterized in that, in the lipid phase, other ionic lipids and/or nonionic lipids are combined with the nonionic amphiphilic lipids of formula I.

**21.** Composition according to one of Claims 19 and 20, characterized in that the lipid phase of the vesicles incorporates additives which enable the permeability of the vesicles to be decreased, and/or charged lipids intended for improving the stability of the vesicles.

**22.** Composition according to Claim 21, characterized in that the additives or charged lipids are chosen from the group composed of sterols and their derivatives, long-chain alcohols and diols, long-chain amines and their quaternary ammonium derivatives, dihydroxyalkylamines, polyoxyethylenated fatty amines, esters of long-chain amino alcohols and their salts and quaternary ammonium derivatives, and phosphoric esters of fatty alcohols.

**23.** Composition according to one of Claims 19 to 22, characterized in that the vesicles contain at least one cosmetic and/or dermopharmaceutical active compound in the lipid phase and/or in the encapsulated phase.

**24.** Composition according to one of Claims 19 to 23, characterized in that the aqueous phase of dispersion of the vesicles contains at least one water-soluble cosmetic and/or dermopharmaceutical cosmetic active compound and/or at least one amphiphilic active compound.

**25.** Compound according to Claim 23, characterized in that the walls of the vesicles contain at least one fat-soluble cosmetic and/or dermopharmaceutical active compound.

**26.** Composition according to one of Claims 19 to 25, characterized in that the aqueous dispersion phase contains a dispersion of droplets of a water-immiscible liquid.

**27.** Composition according to Claim 26, characterized in that the water-immiscible liquid contains at least one fat-soluble cosmetic and/or dermopharmaceutical active compound.

**28.** Composition according to Claim 26, characterized in that the water-immiscible liquid is chosen from the group composed of animal or vegetable oils composed of esters of fatty acids and polyols, natural or synthetic essential oils, halogenated hydrocarbons, silicones, esters of an inorganic acid and an alcohol, ethers and polyethers.

**29.** Composition according to one of Claims 18 to 28, characterized in that the cosmetic and/or dermopharmaceutical active compound is chosen from the group composed of antioxidants or free-radical inhibitors, hydrating or humectant agents, tanning accelerator melanoregulatory agents, depigmenting melanoregulatory agents, skin coloration agents, liporegulators, anti-aging and anti-wrinkle agents, anti-UV agents, keratolytic agents, emollients, anti-inflammatory agents, refreshing agents, cicatrizing agents, vasoprotective agents, antibacterial agents, antifungal agents, insect-repellant agents, antiperspirant agents, deodorant agents, anti-dandruff agents, agents for combating hair loss, hair dyes, hair bleaching agents, reducing agents for permanent waving and skin and hair conditioners.

**30.** Composition according to one of Claims 12 to 28, characterized in that it contains at least one formulation additive having neither cosmetic activity nor dermopharmaceutical activity.

**31.** Composition according to Claim 30, characterized in that the formulation additive is chosen from gelling agents, polymers, preservatives, colorants, opacifiers and perfumes.

**Claims for the following Contracting State : GR**

**1.** Nonionic amphiphilic compounds derived from glycerol, characterized in that they are of the formula (I):

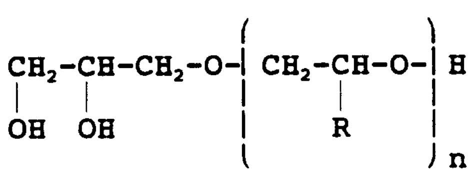

(I)

in which formula:

R represents a radical taken from the group composed of linear or branched $C_4$-$C_{28}$ alkyl or alkenyl radicals and mixtures thereof, or represents a group $-CH_2A$ in which A represents -OR',-SR' or

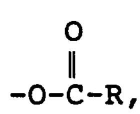

R' representing a saturated or unsaturated hydrocarbon radical, and

- n represents an average statistical value $\bar{n}$ greater than 1 and equal to not more than 6 and, when R is $-CH_2A$, also represents a value equal to 2.

**2.** Compound according to Claim 1, characterized in that the hydrocarbon radical R' is a linear $C_8$-$C_{22}$ alkyl radical, a branched $C_8$-$C_{36}$ alkyl radical, a $C_{18}$ alkenyl radical or an alkylaryl radical having a linear or branched $C_8$-$C_{16}$ alkyl chain.

**3.** Compound according to Claim 2, characterized in that, in the alkylaryl radical, the aryl group is a phenyl radical.

**4.** Compound according to Claim 2, characterized in that the alkenyl radical is a 9-octadecenyl or 9,12-octadecanedienyl radical.

**5.** Compound according to Claim 1, characterized in that the radical R represents a linear $C_{14}$-$C_{18}$ alkyl radical or a group $-CH_2A$ in which A is OR', R' representing a linear $C_{10}$-$C_{18}$ alkyl radical, and n represents an average statistical value $\bar{n}$ greater than 1 and equal to not more than 3 and, when R represents $-CH_2A$, is also equal to 2.

**6.** Process for preparing compounds of formula I according to one of Claims 1 to 5, by a two-stage process with formation of intermediate product, characterized in that :

- in a first stage, isopropylideneglycerol of formula (IV) is reacted in the presence of a basic catalyst with an epoxide of formula (III), in which R has the same meaning as in the formula (I), to obtain one or more intermediate product(s) of formula (II) according to the following reaction scheme:

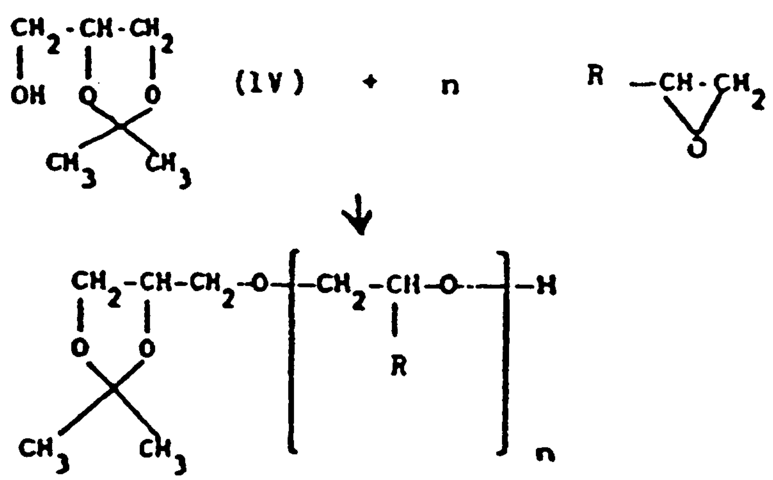

(IV) + n (III)

(II)

R and n having the same meaning as in the formula (I), and

- in a second stage, the intermediate product(s) of formula (II) obtained is/are hydrolyzed and the compound(s) of formula (I) is/are separated from the reaction mixture.

**7.** Process according to Claim 6, characterized in that the basic catalyst used in the first stage is chosen from the group composed of alkali metals, alkali metal hydrides, alkali metal hydroxides, alkali metal alcoholates and alkali metal fluorides.

**8.** Process according to Claim 7, characterized in that the basic catalyst is potassium tertbutylate.

**9.** Process according to one of Claims 6 to 8, characterized in that the amount of basic catalyst used is between 4 and 40 mol % relative to the isopropylideneglycerol of formula (IV).

**10.** Process according to one of Claims 6 to 9, characterized in that, in the first stage, at least a part of the isopropylideneglycerol of formula (IV) and the basic catalyst are mixed under an inert atmosphere, the

mixture is heated to a temperature of between 50 and 150 °C and the epoxide of formula (III) is added.

**11.** Process according to one of Claims 6 to 10, characterized in that, in the second stage, the hydrolysis is performed in the presence of an acid catalyst.

**12.** Process according to one of Claims 8 to 11, characterized in that the hydrolysis is performed in the presence of a solvent.

**13.** Nonionic compounds of formula II

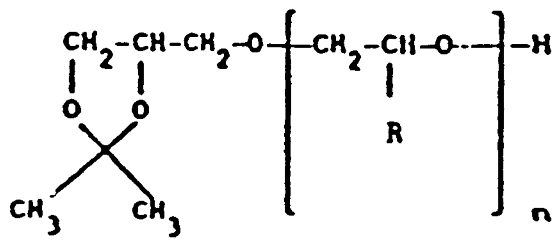

(II)

in which R and n have the same meaning as in Claim 1.

**14.** Cosmetic composition, characterized in that it contains at least one compound of formula I according to one of Claims 1 to 5.

**15.** Composition according to Claim 14, characterized in that it takes the form of an oily composition, a gel, a wax or a water-in-oil or oil-in-water emulsion.

**16.** Composition according to Claim 14, characterized in that it also contains other ionic or nonionic surfactant agents, natural or synthetic, ionic or nonionic polymers, oils or waxes, more or less hydrolyzed proteins, thickeners, pearlescent agents, emollients, hydrating agents, colorants, reducing or oxidizing agents, preservatives, perfumes, anti-UV screening agents, solvents, propellants or pharmaceutical or parapharmaceutical active products.

**17.** Composition according to Claim 14, characterized in that it contains 0.5 to 50 % and preferably from 0.5 to 25 %, of compound of formula (I).

**18.** Composition according to Claim 15, characterized in that it takes the form of an oil-in-water or water-in-oil emulsion and in that it contains, in addition, oils or waxes and, where appropriate, other emulsifying surfactant agents different from those of formula (I).

**19.** Composition according to Claim 18, characterized in that the other emulsifying surfactant agent consists of polyoxyethylenated fatty acids or fatty alcohols, polyglycerol alkyl ethers, esters of fatty acid and sorbitan, polyoxyethylenated or otherwise, esters of fatty acids and sorbitol, polyoxyethylenated or otherwise, polyoxyethylenated castor oil, salts of fatty acids and amines or polyvalent metals, alkyl sulfates, polyoxyethylenated or otherwise, and alkyl phosphates polyoxyethylenated or otherwise.

**20.** Composition according to Claim 14, characterized in that it contains at least one cosmetic and/or dermopharmaceutical active compound.

**21.** Composition according to Claim 14, characterized in that it contains, dispersed in an aqueous phase D, vesicles bounded by one or more lamellae of a lipid phase containing at least one compound of formula I in which R represents a linear $C_{14}$-$C_{18}$ alkyl radical or represents -$CH_2A$, A being OR' and R' representing a linear $C_{10}$-$C_{18}$ alkyl radical, and n represents an average statistical value $\bar{n}$ greater than 1 and equal to not more than 3 and, when R is -$CH_2A$, is also equal to 2.

**22.** Composition according to Claim 21, characterized in that, in the lipid phase, other ionic lipids and/or nonionic lipids are combined with the nonionic amphiphilic lipids of formula I.

**23.** Composition according to one of Claims 21 and 22, characterized in that the lipid phase of the vesicles incorporates additives which enable the permeability of the vesicles to be decreased, and/or charged lipids intended for improving the stability of the vesicles.

**24.** Composition according to Claim 23, characterized in that the additives or charged lipids are chosen from the group composed of sterols and their derivatives, long-chain alcohols and diols, long-chain amines and their quaternary ammonium derivatives, dihydroxyalkylamines, polyoxyethylenated fatty amines, esters of long-chain amino alcohols and their salts and quaternary ammonium derivatives, and phosphoric esters of fatty alcohols.

**25.** Composition according to one of Claims 21 to 24, characterized in that the vesicles contain at least one cosmetic and/or dermopharmaceutical active compound in the lipid phase and/or in the encapsulated phase.

**26.** Composition according to one of Claims 21 to 25, characterized in that the aqueous phase of dispersion of the vesicles contains at least one water-soluble cosmetic and/or dermopharmaceutical cosmetic active compound and/or at least one amphiphilic active compound.

**27.** Compound according to Claim 25, characterized in that the walls of the vesicles contain at least one fat-soluble cosmetic and/or dermopharmaceutical active compound.

**28.** Composition according to one of Claims 21 to 27, characterized in that the aqueous dispersion phase contains a dispersion of droplets of a water-immiscible liquid.

**29.** Composition according to Claim 28, characterized in that the water-immiscible liquid contains at least one fat-soluble cosmetic and/or dermopharmaceutical active compound.

**30.** Composition according to Claim 28, characterized in that the water-immiscible liquid is chosen from the group composed of animal or vegetable oils composed of esters of fatty acids and polyols, natural or synthetic essential oils, halogenated hydrocarbons, silicones, esters of an inorganic acid and an alcohol, ethers and polyethers.

**31.** Composition according to one of Claims 20 to 30, characterized in that the cosmetic and/or dermopharmaceutical active compound is chosen from the group composed of antioxidants or free-radical inhibitors, hydrating or humectant agents, tanning accelerator melanoregulatory agents, depigmenting melanoregulatory agents, skin coloration agents, liporegulators, anti-aging and anti-wrinkle agents, anti-UV agents, keratolytic agents, emollients, anti-inflammatory agents, refreshing agents, cicatrizing agents, vasoprotective agents, antibacterial agents, antifungal agents, insect-repellant agents, anti-perspirant agents, deodorant agents, anti-dandruff agents, agents for combating hair loss, hair dyes, hair bleaching agents, reducing agents for permanent waving and skin and hair conditioners.

**32.** Composition according to one of Claims 14 to 31, characterized in that it contains at least one formulation additive having neither cosmetic activity nor dermopharmaceutical activity.

**33.** Composition according to Claim 32, characterized in that the formulation additive is chosen from gelling agents, polymers, preservatives, colorants, opacifiers and perfumes.

**Patentansprüche**
**Patentansprüche für folgende Vertragsstaaten : AT, BE, CH, DE, DK, FR, GB, IT, LI, LU, NL, SE**

**1.** Nicht-ionische, amphiphile, von Glycerin abgeleitete Verbindungen, dadurch gekennzeichnet, daß sie der Formel (I):

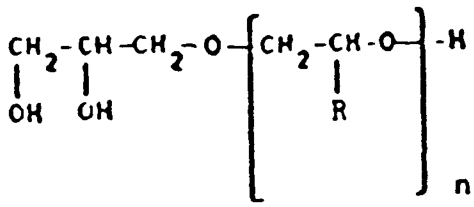

(I)

entsprechen, worin:

- R für einen Rest, der ausgewählt ist unter geradkettigen oder verzweigten $C_4$-$C_{28}$-Alkyl- oder Alkenylresten und ihren Mischungen oder für eine Gruppe -$CH_2$A steht, worin A für -OR', -SR' oder

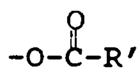

steht, wobei R' einen gesättigten oder ungesättigten Kohlenwasserstoffrest bedeutet und
- n für einen statistischen Mittelwert $\bar{n}$, der größer als 1 und höchstens gleich 6 ist, und, wenn R für -$CH_2$A steht, auch für einen Wert gleich 2 steht.

**2.** Verbindung nach Anspruch 1, dadurch gekennzeichnet, daß der Kohlenwasserstoffrest R' ein linearer $C_8$-$C_{22}$-Alklyrest, ein verzweigter $C_8$-$C_{36}$-Alkylrest, ein $C_{18}$-Alkenylrest oder ein Alkylarylrest mit linearer oder verzweigter $C_8$-$C_{16}$-Alkylkette ist.

**3.** Verbindung nach Anspruch 2, dadurch gekennzeichnet, daß in dem Alkylarylrest die Arylgruppe ein Phenylrest ist.

**4.** Verbindung nach Anspruch 2, dadurch gekennzeichnet, daß der Alkenylrest ein Octadecen-9-yl- oder Octadecandien-9,12-yl-Rest ist.

**5.** Verbindung nach Anspruch 1, dadurch gekennzeichnet, daß der Rest R für einen linearen $C_{14}$-$C_{18}$-Alkylrest oder eine Gruppe -$CH_2$A steht, worin A für OR' steht, wobei R' einen linearen $C_{10}$-$C_{18}$-Alkylrest bedeutet und n einen statistischen Mittelwert n größer als 1 und höchstens gleich 3 bedeutet und, wenn R für -$CH_2$A steht, auch gleich 2 ist.

**6.** Verfahren zur Herstellung der Verbindungen der Formel I nach einem der Ansprüche 1 bis 5 durch ein zweistufiges Verfahren unter Bildung eines Zwischenproduktes, dadurch gekennzeichnet, daß man:
- in einer ersten Stufe Isopropylidenglycerin der Formel (IV) in Anwesenheit eines basischen Katalysators mit einem Epoxid der Formel (III), worin
  R die gleichen Bedeutungen wie in der Formel (I) besitzt, zu einem Zwischenprodukt (zu Zwischenprodukten) der Formel (II) nach dem folgenden Reaktionsschema umsetzt:

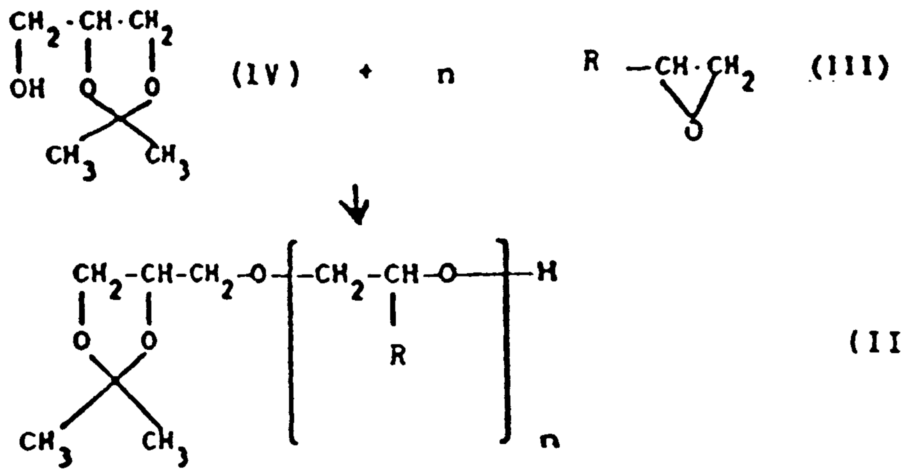


(II)

worin R und n die gleichen Bedeutungen wie in der Formel (I) besitzen und

- in einer zweiten Stufe das erhaltene Zwischenprodukt (die erhaltenen Zwischenprodukte) der Formel (II) hydrolysiert und die Verbindung (en) der Formel (I) von der Reaktionsmischung abtrennt.

**7.** Verfahren nach Anspruch 6, dadurch gekennzeichnet, daß der in der ersten Stufe zur Anwendung kommende basische Katalysator ausgewählt ist unter Alkalimetallen, Alkalimetallhydriden, Alkalihydroxiden, Alkalialkoholaten und Alkalimetallfluoriden.

**8.** Verfahren nach Anspruch 7, dadurch gekennzeichnet, daß der basische Katalysator Kalium-tert-butylat ist.

**9.** Verfahren nach einem der Ansprüche 6 bis 8, dadurch gekennzeichnet, daS die zur Anwendung kommende Menge an basischem Katalysator 4 bis 40 Mol-%, bezogen auf Isopropylidenglycerin der Formel (IV), beträgt.

**10.** Verfahren nach einem der Ansprüche 6 bis 9, dadurch gekennzeichnet, daß man in der ersten Stufe wenigstens einen Teil des Isopropylidenglycerins der Formel (IV) und den basischen Katalysator unter inerter Atmosphäre vermischt, bei einer Temperatur im Bereich von 50 bis 150 °C erhitzt und das Epoxid der Formel (III) zugibt.

**11.** Verfahren nach einem der Ansprüche 6 bis 10, dadurch gekennzeichnet, daß die Hydrolyse in der zweiten Stufe in Anwesenheit eines sauren Katalysators erfolgt.

**12.** Verfahren nach einem der Ansprüche 8 bis 11, dadurch gekennzeichnet, daß man die Hydrolyse in Anwesenheit eines Lösungsmittels durchführt.

**13.** Nicht-ionische Verbindungen der Formel II

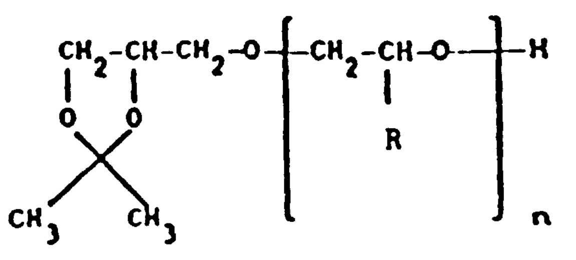

(II)

worin R und n die Anspruch 1 angegebenen Bedeutungen besitzen.

**14.** Kosmetisches und/oder dermopharmazeutisches Mittel, dadurch gekennzeichnet, daß es wenigstens eine Verbindung der Formel I nach einem der Ansprüche 1 bis 5 enthält.

**15.** Mittel nach Anspruch 14, dadurch gekennzeichnet, daß es in Form einer öligen Zusammensetzung, eines Gels, eines Wachses, einer Wasser-in-Öl-Emulsion oder einer Öl-in-Wasser-Emulsion vorliegt.

**16.** Mittel nach Anspruch 14, dadurch gekennzeichnet, daß es auch andere ionische oder nicht-ionische grenzflächenaktive Mittel, natürliche oder synthetische, ionische oder nicht-ionische Polymere, Öle oder Wachse, mehr oder weniger hydrolysierte Proteine, Verdickungsmittel, perlglanzverleihende Mittel, Emollientien, Hydratisierungsmittel, Farbstoffe, Reduktions- oder Oxidationsmittel, Konservierungsmittel, Parfüms, Anti-UV-Filter, Lösungsmittel, Treibmittel oder pharmazeutisch oder parapharmazeutisch aktive Produkte enthält.

**17.** Mittel nach Anspruch 14, dadurch gekennzeichnet, daß es 0,5 bis 50%, vorzugsweise 0,5 bis 25% der Verbindung der Formel (I) enthält.

**18.** Mittel nach Anspruch 15, dadurch gekennzeichnet, daß es in Form einer Öl-in-Wasser- oder Wasser-in-Öl-Emulsion vorliegt und außerdem Öle oder Wachse und gegebenenfalls andere emulgierende grenzflächenaktive Mittel, die von denjenigen der Formel (I) verschieden sind, enthält.

**19.** Mittel nach Anspruch 18, dadurch gekennzeichnet, daß es sich bei dem anderen emulgierenden grenzflächenaktiven Mittel um polyoxyethylenierte Fettsäuren oder Fettalkohole, Polyglycerinalkylether, Ester einer Fettsäure mit gegebenenfalls polyoxyethyleniertem Sorbitan, Ester einer Fettsäure mit gegebenenfalls polyoxyethyleniertem Sorbitol, polyoxyethyleniertes Rizinusöl, Salze aus Fettsäuren und Aminen oder polyvalenten Metallen, gegebenenfalls polyoxyethylenierte Alkylsulfate und gegebenenfalls polyoxyethylenierte Alkylphosphate handelt.

**20.** Mittel nach Anspruch 14, dadurch gekennzeichnet, daß es wenigstens einen kosmetischen und/oder dermopharmazeutischen Wirkstoff enthält.

**21.** Mittel nach Anspruch 14, dadurch gekennzeichnet, daß es, in einer wäßrigen Phase D dispergiert, Vesikel enthält, die durch ein oder mehrere Blättchen einer Lipidphase begrenzt sind, welche wenigstens eine Verbindung der Formel I, worin R für einen linearen $C_{14}$-$C_{18}$-Alkylrest oder für $-CH_2A$ steht, wobei A für OR' steht und R' einen linearen $C_{10}$-$C_{18}$-Alkylrest bedeutet und n einen statistischen Mittelwert $\bar{n}$ größer als 1 und höchstens gleich 3 bedeutet, und wenn R für $-CH_2A$ steht, auch gleich 2 ist, enthalten.

**22.** Mittel nach Anspruch 21, dadurch gekennzeichnet, daß die nicht-ionischen amphiphilen Lipide der Formel I in der Lipidphase mit anderen ionischen Lipiden und/oder nicht-ionischen Lipiden assoziiert sind.

**23.** Mittel nach einem der Ansprüche 21 oder 22, dadurch gekennzeichnet, daß die Lipidphase der Vesikel Additive, die es erlauben, die Permeabilität der Vesikel zu verringern, und/oder geladene Lipide, die zur Verbesserung der Stabilität der Vesikel bestimmt sind, enthält.

**24.** Zusammensetzung nach Anspruch 23, dadurch gekennzeichnet, daß die Additive oder die geladenen Lipide ausgewählt sind unter Sterolen und ihren Derivaten, langkettigen Alkoholen und Diolen, langkettigen Aminen und ihren quaternären Ammoniumderivaten, Dihydroxyalkylaminen, polyoxyethylenierten Fettaminen, Estern von Aminoalkoholen mit langer Kette und ihren Salzen und quaternären Ammoniumderivaten und Fettalkohol-Phosphorsäureestern.

**25.** Mittel nach einem der Ansprüche 21 bis 24, dadurch gekennzeichnet, daß die Vesikel wenigstens einen kosmetischen und/oder dermopharmazeutischen Wirkstoff in der Lipidphase und/oder der eingekapselten Phase enthalten.

**26.** Mittel nach einem der Ansprüche 21 bis 25, dadurch gekennzeichnet, daß die wäßrige Phase der Vesikeldispersion wenigstens einen wasserlöslichen kosmetischen und/oder dermopharmazeutischen Wirkstoff und/oder wenigstens einen amphiphilen Wirkstoff enthält.

**27.** Mittel nach Anspruch 25, dadurch gekennzeichnet, daß die Wände der Vesikel wenigstens einen fettlöslichen kosmetischen und/oder dermopharmazeutischen Wirkstoff enthalten.

**28.** Mittel nach einem der Ansprüche 21 bis 27, dadurch gekennzeichnet, daS die wäßrige Phase der Dispersion eine Dispersion von Tröpfchen einer mit Wasser nicht mischbaren Flüssigkeit enthält.

**29.** Mittel nach Anspruch 28, dadurch gekennzeichnet, daß die mit Wasser nicht mischbare Flüssigkeit wenigstens einen fettlöslichen kosmetischen und/oder dermopharmazeutischen Wirkstoff enthält.

**30.** Mittel nach Anspruch 28, dadurch gekennzeichnet, daß die mit Wasser nicht mischbare Flüssigkeit ausgewählt ist unter tierischen oder pflanzlichen, aus Estern aus Fettsäuren und Polyolen gebildeten Ölen, essentiellen, natürlichen oder synthetischen Ölen, halogenierten Kohlenwasserstoffen, Silikonen, Estern einer Mineralsäure und eines Alkohols, Ethern und Polyethern.

**31.** Mittel nach einem der Ansprüche 20 bis 30, dadurch gekennzeichnet, daß der kosmetische und/oder dermopharmazeutische Wirkstoff ausgewählt ist unter Antioxidantien oder Mitteln gegen freie Radikale, Hydratisierungsmitteln oder Befeuchtungsmitteln, melanoregulatorischen Mitteln zur Beschleunigung der Braünung, melanoregulatorischen depigmentierenden Mitteln, Mitteln zur Färbung der Haut, Liporegulatoren, Mittel gegen das Altern und gegen Falten, Anti-UV-Mitteln, keratolytischen Mitteln, Emollientien, antiinflammatorischen Mitteln, erfrischenden Mitteln, narbenbildenden Mitteln, gefäßschützenden Mitteln, antibakteriellen Mitteln, antifungischen Mitteln, insektifugen Mitteln, Antiperspirantien, Deodorantien, antipellikulären Mitteln, Mitteln gegen Haarausfall, Haarfärbemitteln, Mitteln zur Entfärbung der Haare, Reduktionsmitteln für Dauerwellen, Konditionierungsmitteln für die Haut und die Haare.

**32.** Mittel nach einem der Ansprüche 14 bis 31, dadurch gekennzeichnet, daß es wenigstens ein Formulierungsadditiv mit weder kosmetischer noch dermopharmazeutischer Aktivität enthält.

**33.** Mittel nach Anspruch 32, dadurch gekennzeichnet, daß das Formulierungsadditiv ausgewählt ist unter gelbildenden Mitteln, Polymeren, Konservierungsmitteln, Farbstoffen, opakmachenden Mitteln und Parfüms.

**Patentansprüche für folgenden Vertragsstaat : ES**

**1.** Verfahren zur Herstellung nicht-ionischer, amphiphiler, von Glycerin abgeleiteter Verbindungen durch ein zweistufiges Verfahren unter Bildung eines Zwischenproduktes, dadurch gekennzeichnet, daß man:

- in einer ersten Stufe Isopropylidenglycerin der Formel (IV) in Anwesenheit eines basischen Katalysators mit einem Epoxid der Formel (III), worin R für einen Rest, der ausgewählt ist unter geradkettigen oder verzweigten $C_4$-$C_{28}$-Alkyl- oder Alkenylresten und ihren Mischungen oder für eine Gruppe -$CH_2$A steht, worin A für -OR', -SR' oder

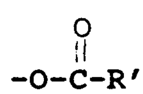

steht, wobei R' einen gesättigten oder ungesättigten Kohlenwasserstoffrest bedeutet, zu einem Zwischenprodukt (zu Zwischenprodukten) der Formel (II) nach dem folgenden Reaktionsschema umsetzt:

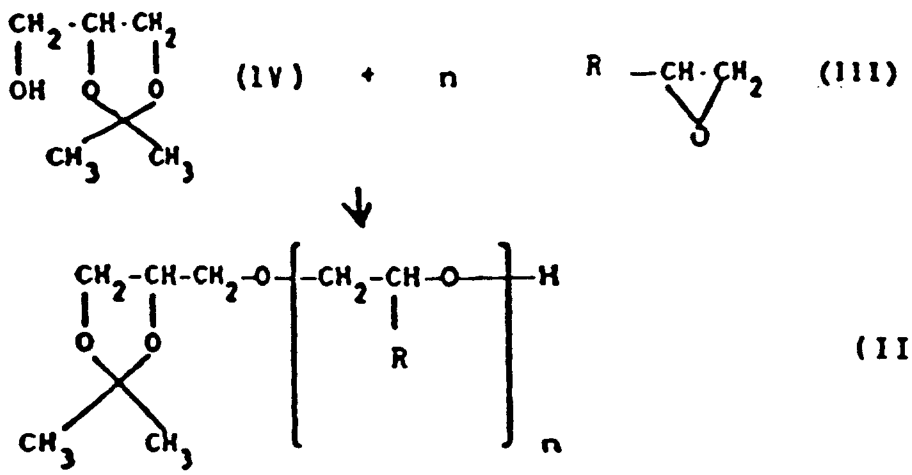

worin R die oben angegebenen Bedeutungen besitzt und n für einen statistischen Mittelwert $\bar{n}$, der größer als 1 und höchstens gleich 6 ist, und, wenn R für $-CH_2A$ steht, auch für einen Wert gleich 2 steht,

- in einer zweiten Stufe das erhaltene Zwischenprodukt (die erhaltenen Zwischenprodukte) der Formel (II) hydrolysiert und die Verbindung(en) der Formel (I):

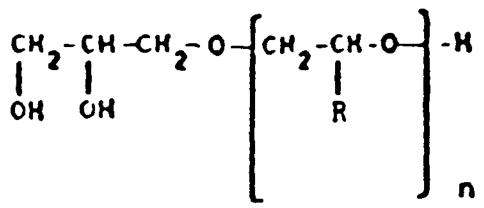

(I)

worin R und n die oben angegebenen Bedeutungen besitzen, von der Reaktionsmischung abtrennt.

**2.** Verfahren nach Anspruch 1, dadurch gekennzeichnet, daß der in der ersten Stufe zur Anwendung kommende basische Katalysator ausgewählt ist unter Alkalimetallen, Alkalimetallhydriden, Alkalihydroxiden, Alkalialkoholaten und Alkalimetallfluoriden.

**3.** Verfahren nach Anspruch 7, dadurch gekennzeichnet, daß der basische Katalysator Kalium-tert-butylat ist.

**4.** Verfahren nach einem der Ansprüche 1 bis 3, dadurch gekennzeichnet, daß die zur Anwendung kommende Menge an basischem Katalysator 4 bis 40 Mol-%, bezogen auf Isopropylidenglycerin der Formel (IV), beträgt.

**5.** Verfahren nach einem der Ansprüche 1 bis 4, dadurch gekennzeichnet, daß man in der ersten Stufe Wenigstens einen Teil des Isopropylidenglycerins der Formel (IV) und den basischen Katalysator unter inerter Atmosphäre vermischt, bei einer Temperatur im Bereich von 50 bis 150 °C erhitzt und das Epoxid der Formel (III) zugibt.

**6.** Verfahren nach einem der Ansprüche 1 bis 5, dadurch gekennzeichnet, daß die Hydrolyse in der zweiten Stufe in Anwesenheit eines sauren Katalysators erfolgt.

**7.** Verfahren nach einem der Ansprüche 3 bis 6, dadurch gekennzeichnet, daß man die Hydrolyse in Anwesenheit eines Lösungsmittels durchführt.

**8.** Kosmetisches Mittel, dadurch gekennzeichnet, daß es wenigstens eine Verbindung der Formel I

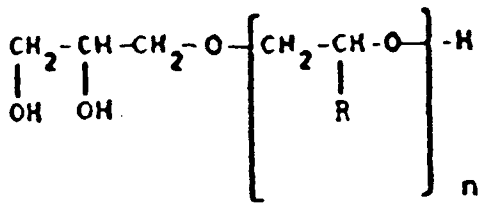

(I)

enthält, worin:

- R für einen Rest, der ausgewählt ist unter geradkettigen oder verzweigten $C_4$-$C_{28}$-Alkyl- oder Alkenylresten und ihren Mischungen oder für eine Gruppe -$CH_2A$ steht, worin A für -OR', -SR' oder

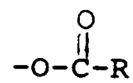

steht, wobei R' einen gesättigten oder ungesättigten Kohlenwasserstoffrest bedeutet und
- n für einen statistischen Mittelwert $\bar{n}$, der größer als 1 und höchstens gleich 6 ist und, wenn R für -$CH_2A$ steht, auch für einen Wert gleich 2 steht.

**9.** Mittel nach Anspruch 8, dadurch gekennzeichnet, daß es wenigstens eine Verbindung der Formel (I) enthält, worin der Kohlenwasserstoffrest R' ein linearer $C_8$-$C_{22}$-Alklyrest, ein Verzweigter $C_8$-$C_{36}$-Alkylrest, ein $C_{18}$-Alkenylrest oder ein Alkylarylrest mit linearer oder verzweigter $C_8$-$C_{16}$-Alkylkette ist.

**10.** Mittel nach Anspruch 9, enthaltend die Verbindung der Formel (I), worin R' für einen Alkylarylrest steht, dadurch gekennzeichnet, daß die Arylgruppe ein Phenylrest ist.

**11.** Mittel nach Anspruch 9, enthaltend eine Verbindung der Formel (I), worin R für einen Alkenylrest steht, dadurch gekennzeichnet, daß der Alkenylrest ein Octadecen-9-yl- oder Octadecandien-9,12-yl-Rest ist.

**12.** Mittel nach Anspruch 8, dadurch gekennzeichnet, daß es wenigstens eine Verbindung der Formel (I) enthält, worin der Rest R für einen linearen $C_{14}$-$C_{18}$-Alkylrest oder eine Gruppe - $CH_2A$ steht, worin A für OR' steht, wobei R' einen linearen $C_{10}$-$C_{18}$-Alkylrest bedeutet und n einen statistischen Mittelwert n größer als 1 und höchstens gleich 3 bedeutet und, wenn R für -$CH_2A$ steht, auch gleich 2 ist.

**13.** Mittel nach Anspruch 8, dadurch gekennzeichnet, daß es in Form einer öligen Zusammensetzung, eines Gels, eines Wachses, einer Wasser-in-Öl-Emulsion oder einer Öl-in-Wasser-Emulsion vorliegt.

**14.** Mittel nach Anspruch 8, dadurch gekennzeichnet, daß es auch andere ionische oder nicht-ionische grenzflächenaktive Mittel, natürliche oder synthetische, ionische oder nicht-ionische Polymere, Öle oder Wachse, mehr oder weniger hydrolysierte Proteine, Verdickungsmittel, perlglanzverleihende Mittel, Emollientien, Hydratisierungsmittel, Farbstoffe, Reduktions- oder Oxidationsmittel, Konservierungsmittel, Parfüms, Anti-UV-Filter, Lösungsmittel, Treibmittel oder pharmazeutisch oder parapharmazeutisch aktive Produkte enthält.

**15.** Mittel nach Anspruch 8, dadurch gekennzeichnet, daß es 0,5 bis 50%, vorzugsweise 0,5 bis 25% der Verbindung der Formel (I) enthält.

**16.** Mittel nach Anspruch 13, dadurch gekennzeichnet, daß es in Form einer Öl-in-Wasser- oder Wasser-in-Öl-Emulsion vorliegt und außerdem Öle oder Wachse und gegebenenfalls andere emulgierende grenzflächenaktive Mittel, die von denjenigen der Formel (I) verschieden sind, enthält.

**17.** Mittel nach Anspruch 16, dadurch gekennzeichnet, daß es sich bei dem anderen emulgierenden grenzflächenaktiven Mittel um polyoxyethylenierte Fettsäuren oder Fettalkohole, polyglycerinalkylether, Ester einer Fettsäure mit gegebenenfalls polyoxyethyleniertem Sorbitan, Ester einer Fettsäure mit gegebenenfalls polyoxyethyleniertem Sorbitol, polyoxyethyleniertes Rizinusöl, Salze aus Fettsäuren und Aminen oder polyvalenten Metallen, gegebenenfalls polyoxyethylenierte Alkylsulfate und gegebenenfalls polyoxyethylenierte Alkylphosphate handelt.

**18.** Mittel nach Anspruch 8, dadurch gekennzeichnet, daß es wenigstens einen kosmetischen und/oder dermopharmazeutischen Wirkstoff enthält.

**19.** Mittel nach Anspruch 8, dadurch gekennzeichnet, daß es, in einer wäßrigen Phase D dispergiert, Vesikel enthält, die durch ein oder mehrere Blättchen einer Lipidphase begrenzt sind, welche wenigstens eine Verbindung der Formel I, worin R für einen linearen $C_{14}$-$C_{18}$-Alkylrest oder für $-CH_2A$ steht, wobei A für OR' steht und R' einen linearen $C_{10}$-$C_{18}$-Alkylrest bedeutet und n einen statistischen Mittelwert $\bar{n}$ größer als 1 und höchstens gleich 3 bedeutet, und wenn R für $-CH_2A$ steht, auch gleich 2 ist, enthalten.

**20.** Mittel nach Anspruch 19, dadurch gekennzeichnet, daß die nicht-ionischen amphiphilen Lipide der Formel I in der Lipidphase mit anderen ionischen Lipiden und/oder nicht-ionischen Lipiden assoziiert sind.

**21.** Mittel nach einem der Ansprüche 19 oder 20, dadurch gekennzeichnet, daß die Lipidphase der Vesikel Additive, die es erlauben, die Permeabilität der Vesikel zu verringern, und/oder geladene Lipide, die zur Verbesserung der Stabilität der Vesikel bestimmt sind, enthält.

**22.** Zusammensetzung nach Anspruch 21, dadurch gekennzeichnet, daß die Additive oder die geladenen Lipide ausgewählt sind unter Sterolen und ihren Derivaten, langkettigen Alkoholen und Diolen, langkettigen Aminen und ihren quaternären Ammoniumderivaten, Dihydroxyalkylaminen, polyoxyethylenierten Fettaminen, Estern von Aminoalkoholen mit langer Kette und ihren Salzen und quaternären Ammoniumderivaten und Fettalkohol-Phosphorsäureestern.

**23.** Mittel nach einem der Ansprüche 19 bis 22, dadurch gekennzeichnet, daß die Vesikel wenigstens einen kosmetischen und/oder dermopharmazeutischen Wirkstoff in der Lipidphase und/oder der eingekapselten Phase enthalten.

**24.** Mittel nach einem der Ansprüche 19 bis 23, dadurch gekennzeichnet, daß die wäßrige Phase der Vesikeldispersion wenigstens einen wasserlöslichen kosmetischen und/oder dermopharmazeutischen Wirkstoff und/oder wenigstens einen amphiphilen Wirkstoff enthält.

**25.** Mittel nach Anspruch 23, dadurch gekennzeichnet, daß die Wände der Vesikel wenigstens einen fettlöslichen kosmetischen und/oder dermopharmazeutischen Wirkstoff enthalten.

**26.** Mittel nach einem der Ansprüche 19 bis 25, dadurch gekennzeichnet, daß die wäßrige Phase der Dispersion eine Dispersion von Tröpfchen einer mit Wasser nicht mischbaren Flüssigkeit enthält.

**27.** Mittel nach Anspruch 26, dadurch gekennzeichnet, daß die mit Wasser nicht mischbare Flüssigkeit wenigstens einen fettlöslichen kosmetischen und/oder dermopharmazeutischen Wirkstoff enthält.

**28.** Mittel nach Anspruch 26, dadurch gekennzeichnet, daß die mit Wasser nicht mischbare Flüssigkeit ausgewählt ist unter tierischen oder Pflanzlichen, aus Estern aus Fettsäuren und Polyolen gebildeten Ölen, essentiellen, natürlichen oder synthetischen Ölen, halogenierten Kohlenwasserstoffen, Silikonen, Estern einer Mineralsäure und eines Alkohols, Ethern und Polyethern.

**29.** Mittel nach einem der Ansprüche 18 bis 28, dadurch gekennzeichnet, daß der kosmetische und/oder dermopharmazeutische Wirkstoff ausgewählt ist unter Antioxidantien oder Mitteln gegen freie Radikale, Hydratisierungsmitteln oder Befeuchtungsmitteln, melanoregulatorischen Mitteln zur Beschleunigung der Braünung, melanoregulatorischen depigmentierenden Mitteln, Mitteln zur Färbung der Haut, Liporegulatoren, Mittel gegen das Altern und gegen Falten, Anti-UV-Mitteln, keratolytischen Mitteln, Emollientien, antiinflammatorischen Mitteln, erfrischenden Mitteln, narbenbildenden Mitteln, gefäßschützenden Mitteln, antibakteriellen Mitteln, antifungischen Mitteln, insektifugen Mitteln, Antiperspirantien, Deodorantien, antipellikulären Mitteln, Mitteln gegen Haarausfall, Haarfärbemitteln, Mitteln zur Entfärbung der Haare, Reduktionsmitteln für Dauerwellen, Konditionierungsmitteln für die Haut und die Haare.

**30.** Mittel nach einem der Ansprüche 12 bis 28, dadurch gekennzeichnet, daß es wenigstens ein formulierungsadditiv mit weder kosmetischer noch dermopharmazeutischer Aktivität enthält.

**31.** Mittel nach Anspruch 30, dadurch gekennzeichnet, daß das Formulierungsadditiv ausgewählt ist unter gelbildenden Mitteln, Polymeren, Konservierungsmitteln, Farbstoffen, opakmachenden Mitteln und Parfüms.

**Patentansprüche für folgenden Vertragsstaat : GR**

**1.** Nicht-ionische, amphiphile, von Glycerin abgeleitete Verbindungen, dadurch gekennzeichnet, daß sie der Formel (I):

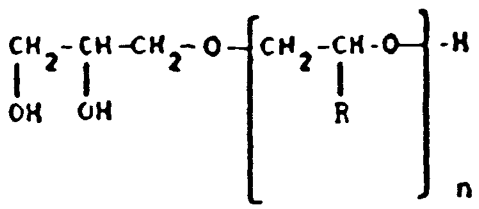

(I)

entsprechen, worin:

- R für einen Rest, der ausgewählt ist unter geradkettigen oder verzweigten $C_4$-$C_{28}$-Alkyl- oder Alkenylresten und ihren Mischungen oder für eine Gruppe -$CH_2$A steht, worin A für -OR', -SR' oder

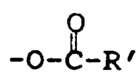

steht, wobei R' einen gesättigten oder ungesättigten Kohlenwasserstoffrest bedeutet und
- n für einen statistischen Mittelwert $\bar{n}$, der größer als 1 und höchstens gleich 6 ist, und, wenn R für -$CH_2$A steht, auch für einen Wert gleich 2 steht.

**2.** Verbindung nach Anspruch 1, dadurch gekennzeichnet, daß der Kohlenwasserstoffrest R' ein linearer $C_8$-$C_{22}$-Alklyrest, ein verzweigter $C_8$-$C_{36}$-Alkylrest, ein $C_{18}$-Alkenylrest oder ein Alkylarylrest mit linearer oder verzweigter $C_8$-$C_{16}$-Alkylkette ist.

**3.** Verbindung nach Anspruch 2, dadurch gekennzeichnet, daß in dem Alkylarylrest die Arylgruppe ein Phenylrest ist.

**4.** Verbindung nach Anspruch 2, dadurch gekennzeichnet, daß der Alkenylrest ein Octadecen-9-yl- oder Octadecandien-9,12-yl-Rest ist.

**5.** Verbindung nach Anspruch 1, dadurch gekennzeichnet, daß der Rest R für einen linearen $C_{14}$-$C_{18}$-Alkylrest oder eine Gruppe -$CH_2$A steht, worin A für OR' steht, wobei R' einen linearen $C_{10}$-$C_{18}$-Alkylrest bedeutet und n einen statistischen Mittelwert n größer als 1 und höchstens gleich 3 bedeutet und, wenn R für -$CH_2$A steht, auch gleich 2 ist.

**6.** Verfahren zur Herstellung der Verbindungen der Formel I nach einem der Ansprüche 1 bis 5 durch ein zweistufiges Verfahren unter Bildung eines Zwischenproduktes, dadurch gekennzeichnet, daß man:

- in einer ersten Stufe Isopropylidenglycerin der Formel (IV) in Anwesenheit eines basischen Katalysators mit einem Epoxid der Formel (III), worin

R die gleichen Bedeutungen wie in der Formel (I) besitzt, zu einem Zwischenprodukt (zu Zwischenprodukten) der Formel (II) nach dem folgenden Reaktionsschema umsetzt:

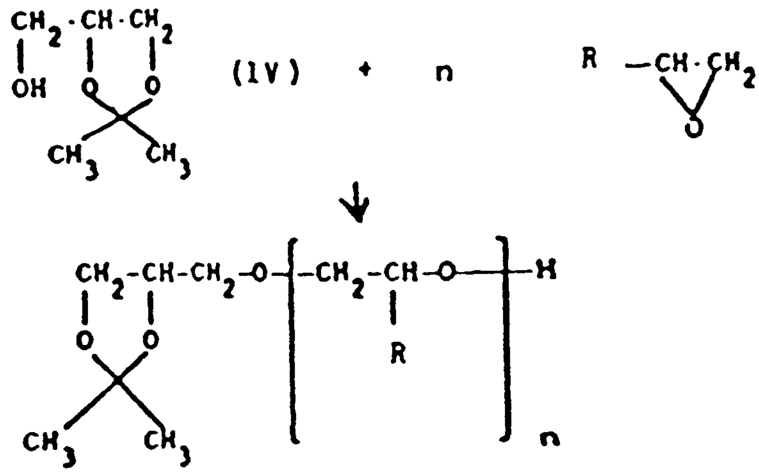

(IV) + n (III)

(II)

worin R und n die gleichen Bedeutungen wie in der Formel (I) besitzen und

- in einer zweiten Stufe das erhaltene Zwischenprodukt (die erhaltenen Zwischenprodukte) der Formel (II) hydrolysiert und die Verbindung (en) der Formel (I) von der Reaktionsmischung abtrennt.

**7.** Verfahren nach Anspruch 6, dadurch gekennzeichnet, daß der in der ersten Stufe zur Anwendung kommende basische Katalysator ausgewählt ist unter Alkalimetallen, Alkalimetallhydriden, Alkalihydroxiden, Alkalialkoholaten und Alkalimetallfluoriden.

**8.** Verfahren nach Anspruch 7, dadurch gekennzeichnet, daß der basische Katalysator Kalium-tert-butylat ist.

**9.** Verfahren nach einem der Ansprüche 6 bis 8, dadurch gekennzeichnet, daß die zur Anwendung kommende Menge an basischem Katalysator 4 bis 40 Mol-%, bezogen auf Isopropylidenglycerin der Formel (IV), beträgt.

**10.** Verfahren nach einem der Ansprüche 6 bis 9, dadurch gekennzeichnet, daß man in der ersten Stufe wenigstens einen Teil des Isopropylidenglycerins der Formel (IV) und den basischen Katalysator unter inerter Atmosphäre vermischt, bei einer Temperatur im Bereich von 50 bis 150 °C erhitzt und das Epoxid der Formel (III) zugibt.

**11.** Verfahren nach einem der Ansprüche 6 bis 10, dadurch gekennzeichnet, daß die Hydrolyse in der zweiten Stufe in Anwesenheit eines sauren Katalysators erfolgt.

**12.** Verfahren nach einem der Ansprüche 8 bis 11, dadurch gekennzeichnet, daß man die Hydrolyse in Anwesenheit eines Lösungsmittels durchführt.

**13.** Nicht-ionische Verbindungen der Formel II

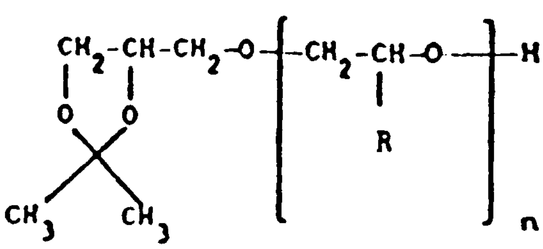

(II)

worin R und n die Anspruch 1 angegebenen Bedeutungen besitzen.

**14.** Kosmetisches Mittel, dadurch gekennzeichnet, daß es wenigstens eine Verbindung der Formel I nach einem der Ansprüche 1 bis 5 enthält.

**15.** Mittel nach Anspruch 14, dadurch gekennzeichnet, daß es in Form einer öligen Zusammensetzung, eines Gels, eines Wachses, einer Wasser-in-Öl-Emulsion oder einer Öl-in-Wasser-Emulsion vorliegt.

**16.** Mittel nach Anspruch 14, dadurch gekennzeichnet, daß es auch andere ionische oder nicht-ionische grenzflächenaktive Mittel, natürliche oder synthetische, ionische oder nicht-ionische Polymere, Öle oder Wachse, mehr oder weniger hydrolysierte Proteine, Verdickungsmittel, perlglanzverleihende Mittel, Emollientien, Hydratisierungsmittel, Farbstoffe, Reduktions- oder Oxidationsmittel, Konservierungsmittel, Parfüms, Anti-UV-Filter, Lösungsmittel, Treibmittel oder pharmazeutisch oder parapharmazeutisch aktive Produkte enthält.

**17.** Mittel nach Anspruch 14, dadurch gekennzeichnet, daß es 0,5 bis 50%, vorzugsweise 0,5 bis 25% der Verbindung der Formel (I) enthält.

**18.** Mittel nach Anspruch 15, dadurch gekennzeichnet, daß es in Form einer Öl-in-Wasser- oder Wasser-in-Öl-Emulsion vorliegt und außerdem Öle oder Wachse und gegebenenfalls andere emulgierende grenzflächenaktive Mittel, die von denjenigen der Formel (I) verschieden sind, enthält.

**19.** Mittel nach Anspruch 18, dadurch gekennzeichnet, daß es sich bei dem anderen emulgierenden grenzflächenaktiven Mittel um polyoxyethylenierte Fettsäuren oder Fettalkohole, Polyglycerinalkylether, Ester einer Fettsäure mit gegebenenfalls polyoxyethyleniertem Sorbitan, Ester einer Fettsäure mit gegebenenfalls polyoxyethyleniertem Sorbitol, polyoxyethyleniertes Rizinusöl, Salze aus Fettsäuren und Aminen oder polyvalenten Metallen, gegebenenfalls polyoxyethylenierte Alkylsulfate und gegebenenfalls polyoxyethylenierte Alkylphosphate handelt.

**20.** Mittel nach Anspruch 14, dadurch gekennzeichnet, daß es wenigstens einen kosmetischen und/oder dermopharmazeutischen Wirkstoff enthält.

**21.** Mittel nach Anspruch 14, dadurch gekennzeichnet, daß es, in einer wäßrigen Phase D dispergiert, Vesikel enthält, die durch ein oder mehrere Blättchen einer Lipidphase begrenzt sind, welche wenigstens eine Verbindung der Formel I, worin R für einen linearen $C_{14}$-$C_{18}$-Alkylrest oder für $-CH_2A$ steht, wobei A für OR' steht und R' einen linearen $C_{10}$-$C_{18}$-Alkylrest bedeutet und n einen statistischen Mittelwert $\bar{n}$ größer als 1 und höchstens gleich 3 bedeutet, und wenn R für $-CH_2A$ steht, auch gleich 2 ist, enthalten.

**22.** Mittel nach Anspruch 21, dadurch gekennzeichnet, daß die nicht-ionischen amphiphilen Lipide der Formel I in der Lipidphase mit anderen ionischen Lipiden und/oder nicht-ionischen Lipiden assoziiert sind.

**23.** Mittel nach einem der Ansprüche 21 oder 22, dadurch gekennzeichnet, daß die Lipidphase der Vesikel Additive, die es erlauben, die Permeabilität der Vesikel zu verringern, und/oder geladene Lipide, die zur Verbesserung der Stabilität der Vesikel bestimmt sind, enthält.

**24.** Zusammensetzung nach Anspruch 23, dadurch gekennzeichnet, daß die Additive oder die geladenen Lipide ausgewählt sind unter Sterolen und ihren Derivaten, langkettigen Alkoholen und Diolen, langkettigen Aminen und ihren quaternären Ammoniumderivaten, Dihydroxyalkylaminen, polyoryethylenierten Fettaminen, Estern von Aminoalkoholen mit langer Kette und ihren Salzen und guaternären Ammoniumderivaten und Fettalkohol-Phosphorsäureestern.

**25.** Mittel nach einem der Ansprüche 21 bis 24, dadurch gekennzeichnet, daß die Vesikel wenigstens einen kosmetischen und/oder dermopharmazeutischen Wirkstoff in der Lipidphase und/oder der eingekapselten Phase enthalten.

**26.** Mittel nach einem der Ansprüche 21 bis 25, dadurch gekennzeichnet, daß die wäßrige Phase der Vesikeldispersion wenigstens einen wasserlöslichen kosmetischen und/oder dermopharmazeutischen Wirkstoff und/oder wenigstens einen amphiphilen Wirkstoff enthält.

**27.** Mittel nach Anspruch 25, dadurch gekennzeichnet, daß die Wände der Vesikel wenigstens einen fettlöslichen kosmetischen und/oder dermopharmazeutischen Wirkstoff enthalten.

**28.** Mittel nach einem der Ansprüche 21 bis 27, dadurch gekennzeichnet, daß die wäßrige Phase der Dispersion eine Dispersion von Tröpfchen einer mit Wasser nicht mischbaren Flüssigkeit enthält.

**29.** Mittel nach Anspruch 28, dadurch gekennzeichnet, daß die mit Wasser nicht mischbare Flüssigkeit wenigstens einen fettlöslichen kosmetischen und/oder dermopharmazeutischen Wirkstoff enthält.

**30.** Mittel nach Anspruch 28, dadurch gekennzeichnet, daß die mit Wasser nicht mischbare Flüssigkeit ausgewählt ist unter tierischen oder pflanzlichen, aus Estern aus Fettsäuren und Polyolen gebildeten Ölen, essentiellen, natürlichen oder synthetischen Ölen, halogenierten Kohlenwasserstoffen, Silikonen, Estern einer Mineralsäure und eines Alkohols, Ethern und Polyethern.

**31.** Mittel nach einem der Ansprüche 20 bis 30, dadurch gekennzeichnet, daß der kosmetische und/oder dermopharmazeutische Wirkstoff ausgewählt ist unter Antioxidantien oder Mitteln gegen freie Radikale, Hydratisierungsmitteln oder Befeuchtungsmitteln, melanoregulatorischen Mitteln zur Beschleunigung der Braünung, melanoregulatorischen depigmentierenden Mitteln, Mitteln zur Färbung der Haut, Liporegulatoren, Mittel gegen das Altern und gegen Falten, Anti-UV-Mitteln, keratolytischen Mitteln, Emollientien, antiinflammatorischen Mitteln, erfrischenden Mitteln, narbenbildenden Mitteln, gefäßschützenden Mitteln, antibakteriellen Mitteln, antifungischen Mitteln, insektifugen Mitteln, Antiperspirantien, Deodorantien, antipellikulären Mitteln, Mitteln gegen Haarausfall, Haarfärbemitteln, Mitteln zur Entfärbung der Haare, Reduktionsmitteln für Dauerwellen, Konditionierungsmitteln für die Haut und die Haare.

**32.** Mittel nach einem der Ansprüche 14 bis 31, dadurch gekennzeichnet, daß es wenigstens ein Formulierungsadditiv mit weder kosmetischer noch dermopharmazeutischer Aktivität enthält.

**33.** Mittel nach Anspruch 32, dadurch gekennzeichnet, daß das Formulierungsadditiv ausgewählt ist unter gelbildenden Mitteln, Polymeren, Konservierungsmitteln, Farbstoffen, opakmachenden Mitteln und Parfüms.